(19) Europäisches Patentamt
European Patent Office
Office européen des brevets

(11) EP 2 837 696 B1

(12) EUROPEAN PATENT SPECIFICATION

(45) Date of publication and mention of the grant of the patent:
**31.01.2018 Bulletin 2018/05**

(21) Application number: **13776121.9**

(22) Date of filing: **08.04.2013**

(51) Int Cl.:
***C12Q 1/68*** *(2018.01)* ***C12N 15/11*** *(2006.01)*
***C12Q 1/00*** *(2006.01)*

(86) International application number:
**PCT/KR2013/002895**

(87) International publication number:
**WO 2013/154304 (17.10.2013 Gazette 2013/42)**

(54) **HIGH-SENSITIVITY NUCLEIC ACID PREPARATION METHODS FOR THE DETECTION OF NUCLEIC ACID BY NUCLEIC ACID POLYMERASE**

VERFAHREN ZUR HERSTELLUNG VON NUCLEINSÄUREN MIT HOHER SENSIBILITÄT FÜR DIE DETEKTION VON NUCLEINSÄUREN DURCH EINE NUCLEINSÄURE-POLYMERASE

MÉTHODES DE PRÉPARATION D’ACIDES NUCLÉIQUES DE HAUTES SENSIBILITÉS POUR LA DÉTECTION D’ACIDES NUCLÉIQUES PAR UNE POLYMÉRASE À ACIDES NUCLÉIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.04.2012 KR 20120036978**
**24.08.2012 KR 20120093222**

(43) Date of publication of application:
**18.02.2015 Bulletin 2015/08**

(73) Proprietor: **Bioneer Corporation**
**Daejeon 306-220 (KR)**

(72) Inventors:
- **PARK, Han Oh**
  **Daejeon305-761 (KR)**
- **LEE, Jun Hee**
  **Daejeon 301-810 (KR)**
- **KIM, Hyun Seo**
  **Daejeon 300-801 (KR)**
- **CHOI, Sora**
  **Yeongi-gun**
  **Chungcheongnam-do 339-833 (KR)**
- **KIM, Jong Hoon**
  **Daejeon 305-743 (KR)**

(74) Representative: **Ladendorf, Oliver**
**Kraus & Weisert**
**Patentanwälte PartGmbB**
**Thomas-Wimmer-Ring 15**
**80539 München (DE)**

(56) References cited:
**WO-A1-99/19341**
**WO-A2-2005/033328**
**US-A- 6 017 702**
**US-A- 6 017 702**
**US-A1- 2002 187 477**
**US-A1- 2007 264 655**

- **SANGER F ET AL: "DNA sequencing with chain-terminating inhibitors", PROCEEDINGS OF THE NATIONAL ACADEMY OF SCIENCES, NATIONAL ACADEMY OF SCIENCES, US, vol. 74, no. 12, 1 January 1977 (1977-01-01), pages 5463-5467, XP008154983, ISSN: 0027-8424**
- **KASAHARA Y ET AL: "Effect of 3@?-end capping of aptamer with various 2@?,4@?-bridged nucleotides: Enzymatic post-modification toward a practical use of polyclonal aptamers", BIOORGANIC & MEDICINAL CHEMISTRY LETTERS, PERGAMON, AMSTERDAM, NL, vol. 20, no. 5, 1 March 2010 (2010-03-01), pages 1626-1629, XP026911320, ISSN: 0960-894X, DOI: 10.1016/J.BMCL.2010.01.028 [retrieved on 2010-02-17]**
- **FINN P J ET AL: "Synthesis and application of charge-modified dye-labeled dideoxynucleoside-5’-triphosphates to direct-load DNA sequencing--", NUCLEIC ACIDS RESEARCH, INFORMATION RETRIEVAL LTD, GB, vol. 30, no. 13, 1 January 2002 (2002-01-01), pages 2877-2885, XP002243107, ISSN: 0305-1048, DOI: 10.1093/NAR/GKF387**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 837 696 B1

- ANDREW J H SMITH: "Nucleic Acids Research The use of exonuclease III for preparing single stranded DNA for use as a template in the chain terminator sequencing method", NUCLEIC ACIDS RES., vol. 6, no. 3, 1 March 1979 (1979-03-01), pages 831-848, XP055166475,
- PENG HUANG ET AL: "Selective Action of 3'-Azido-3'-deoxythymidine 5'-Triphosphate on Viral Reverse Transcriptases and Human DNA Polymerases*", THE JOURNAL OR BIOL.OCICAL CHEMISTRY, vol. 265, 15 July 1990 (1990-07-15), XP055166476,
- FINN, P. J. ET AL.: 'Synthesis and application of charge-modified dye-labeled dideoxynucleoside-5'-triphosphates to 'direct-load' DNA sequencing' NUCLEIC ACIDS RES. vol. 30, no. 13, 01 July 2002, pages 2877 - 2885, XP002243107
- SMITH, A. J. H. ET AL.: 'The use of exonuclease III for preparing single stranded DNA for use as a template in the chain terminator sequencing method' NUCLEIC ACIDS RES. vol. 6, no. 3, March 1979, pages 831 - 848, XP055166475
- HUANG, P. ET AL.: 'Selective action of 3'-azido-3'-deoxythymidine 5'-triphosphate on viral reverse transcriptases and human DNA polymerases' J. BIOL. CHEM. vol. 265, no. 20, 15 July 1990, pages 11914 - 11918, XP055166476

## Description

[Technical Field]

The present invention relates to a method for treating a nucleic acid before amplifying the nucleic acid using a nucleic acid polymerization reaction to prevent a non-specific priming of the nucleic acid..

[Background Art]

Various methods for detecting a nucleic acid using nucleic acid polymerase have been used for various inspections such as tests for viruses, pathogens, carcinogenic gene, and the like, with high sensitivity and specificity. High sensitivity might be achieved by amplifying the number of target nucleic acid to be hundreds of millions or more using nucleic acid polymerase for detection. High specificity might be achieved by using a primer specifically hybridized to the target nucleic acid to selectively perform a nucleic acid polymerization reaction by priming through selective hybridization to the target nucleic acid at a hybridization temperature. However, in the detection methods with various nucleic acid polymerases such as a PCR reaction, a real time quantitative PCR reaction, and the like, for detecting a trace of nucleic acid, non-specific priming and a subsequent polymerization reaction act as a main factor deteriorating a detection limit. The non-specific amplification is caused because the added primer is hybridized to the target nucleic acid for a priming reaction, and single-stranded nucleic acids which are competitively present in many numbers are non-specifically and partially hybridized for priming, such that nucleic acid polymerization reactions competitively occur.

When the non-specific nucleic acid polymerization reaction occurs many times, since elements required for the nucleic acid polymerization reaction are consumed before a desired target nucleic acid is amplified, the nucleic acid polymerization reaction does not progress any further, such that amplification and detection of the target nucleic acid end in failure. In particular, the non-specific nucleic acid polymerization reaction is seriously affected when the number of target nucleic acids is significantly small, and the number of non-target nucleic acids including a large number of single-stranded nucleic acids is significantly large. For example, in the case of the real time quantitative PCR using a probe, as a ratio of the non-target nucleic acid becomes gradually increased, a delta Rn value of the real time quantitative PCR curve becomes gradually decreased, and eventually, the target is not detectable over a certain amount.

Detection for RNA mostly including the single-stranded nucleic acids is generally performed with a smaller sensitivity as compared to detection for DNA. In general, a method for detecting RNA is performed by reverse transcriptase (RT) reaction, followed by PCR reaction. For the reaction with high sensitivity, the RT reaction is firstly performed in one reactor, and after the RT reaction is finished, the PCR reaction is performed with the RT reaction product in a separate reactor.

However, in this case, since a reaction tube needs to be re-opened for adding a solution, which is inconvenient, and has a high possibility of being contaminated when opening the reaction tube and performing an operation, a reverse transcriptase-polymerase chain reaction (RT-PCR) method generally including reverse transcriptase (RT) in one tube, followed by PCR reaction, has been mainly used. However, in this case, there is a problem that sensitivity is largely deteriorated as compared to a case when the reactions are separately performed.

The reverse transcriptase-polymerase chain reaction (RT-PCR) requires a primer at the time of performing the reverse transcriptase reaction, wherein there are a method of using a random primer and a method of using an anti-sense primer which is a target-specific primer.

The method of using the random primer is capable of preparing cDNA with respect to all nucleic acids, and therefore, has been mainly used to prepare a cDNA library or cDNA for analyzing transcriptome. However, in the case of detecting a trace of RNA, since cDNAs with respect to all RNAs are synthesized to prepare a number of non-target cDNAs, the amount of non-target DNA is excessively increased, such that it is difficult to detect the target as described above. Detection requiring high sensitivity such as RNA virus diagnostic kit as described above generally employs a method for using an anti-sense primer complementary to a target RNA.

The target-specific anti-sense primer is generally capable of increasing the detection limit. However, even though the target-specific primer is used, since the non-specific priming occurred at a temperature lower than a hybridization temperature of the primer in mixing reaction solutions containing a reverse transcriptase having high activity at room temperature with RNAs, and the subsequent reverse transcriptase reaction, are not preventable, a problem that a number of non-specific cDNAs are prepared occurs.

A technology developed so as to overcome the above-described problem and detect the trace of nucleic acid is a hot start PCR reaction. The hot start PCR reaction is a method of including a sample mixing process to control so as not to initiate the PCR reaction at a low temperature and then, activating the PCR reaction only over the hybridization temperature of the target-specific primer.

In this method, the PCR reaction initiates only after reaching a temperature when the primer achieves complete hybridization to the target nucleic acid, thereby preventing the progress of the PCR reaction with non-specific priming

at a temperature lower than the hybridization temperature of the primer. As a specific method, the hot start PCR method was developed, wherein pyrophosphate which is a polymerase inhibitor is contained in a reaction solution, and pyrophosphatase decomposing pyrophosphate is additionally contained in the reaction solution to eliminate pyrophosphate by reaction at the hybridization temperature of the primer for a predetermined time, such that the PCR reaction is then initiated, whereby non-specific nucleic acid synthesis may be inhibited at a low temperature to detect a trace of nucleic acid. The hot start PCR method was described in Korean Patent No. 10-1098764 filed by the same applicant herein.

However, in the case in which the target RNA is contained in a trace amount, even though the reaction is performed by the anti-sense primer or the hot start reverse transcriptase reaction, synthesis of only cDNA with respect to the target RNA is not capable of being actually performed. The reason is that various RNAs rather than the primer are capable of hybridized at a temperature when the anti-sense primer is hybridized. The various non-target RNAs may function as the primer for the reverse transcriptase reaction, and eventually, may be hybridized to the target nucleic acid and function as the primer to prepare various cDNAs.

In particular, in the case of performing the reverse transcriptase reaction using samples containing a large amount of RNAs each having a short length of 100 bp or less such as RNA extracted from blood serum or paraffin block tissue as a template, a number of RNAs having a slightly longer length than that of the primer are contained, such that various RNAs are primed at the reverse transcriptase reaction temperature to prepare extremely various numbers of cDNAs. The cDNA participates in the PCR reaction, producing various non-specific amplified products.

This phenomenon is a serious problem when performing an inspection targeting bio-samples containing a trace of cancer cell or RNA virus. To detect the target RNA, nucleic acids need to be extracted from the bio-sample in an amount as large as possible, wherein the extracted amount of RNA is increased in proportion with the amount of the bio-sample, an amount of the non-target RNA is inevitably increased, and in the end, innumerable cDNAs are prepared by the non-specific reverse transcriptase reaction. In addition, among the cDNAs, cDNAs having base sequence which is partially similar to the target-specific primer are included to be non-specifically amplified in the subsequent PCR reaction step, such that before the amplification of the target nucleic acids, the non-target nucleic acids are amplified to complete the reaction, eventually, which becomes the main reasons of precluding amplification and detection of RNAs of the trace of cancer cell or virus.

In order to solve the above-described problems, various attempts are being made.

First, there are attempts to increase the detection limit of the specific target by more specifically hybridizing the primer. A method of substituting 5'-terminal of the primer with LNA to be more strongly hybridized to the target, consequently, reducing the non-specific amplification was developed (Quantification of low-expressed mRNA using 5' LNA-containing real-time PCR primers. Malgoyre A, Banzet S, Mouret C, Bigard AX, Peinnequin A. Biochem Biophys Res Commun. 2007 Mar 2;354(1) :246-52. Epub 2007 Jan 3.).

Second, when performing the RT reaction in the RT reaction step, in particular, at a low temperature, the polymerization progresses by partial hybridization between the primers, such that a dimer may be formed, which is one of main reasons rapidly deteriorating sensitivity in the RT reaction. In order to solve the problem, technologies that the primer is not partially hybridized at a low temperature as much as possible have been developed. A primer which is elongated so that 5 bp bases of 5' base sequences form hairpins was developed (Development of a highly sensitive real-time one step RT-PCR combined complementary locked primer technology and conjugated minor groove binder probe, JiYoung Hong, Byunghak Kang, Ahyoun Kim, Seoyeon Hwang, Jinhee Ahn, Sunhwa Lee, Jonghyen Kim, Jae-Hak Park, Doo-Sung Cheon Virol J. 2011; 8: 330. Published online 2011 June 29. doi: 10.1186/1743-422X-8-330).

However, since the rock primer method has high hybridization temperature by additionally added base sequences, non-specific hybridization to a non-specific target having similar base sequences may be induced, and efficiency may be slightly deteriorated by the hairpin structure in the hybridization step.

In addition, it was announced that when cDNA is synthesized at a high temperature such as 70°C by using the primer which is hybridized at a high temperature and AMV reverse transcriptase operated even at a high temperature, the primer may be more specifically amplified (High temperature cDNA synthesis by AMV reverse transcriptase improves the specificity of PCR. Fuchs B, Zhang K, Rock MG, Bolander ME, Sarkar G. Mol Biotechnol. 1999 Oct; 12(3):237-40.). In the multiplex RT-PCR reaction, specificity is more important.

The multiplex one tube RT-PCR method is a method of using phosphotriester decomposed by heat, wherein the primer is initially blocked and then sequentially hydrolyzed by heat and operated as a primer (BMC Mol Biol. 2009 Dec 30;10:113. Selective control of primer usage in multiplex one-step reverse transcription PCR. Hidalgo Ashrafi E, Yee J, Paul N.). By using the method, the non-specific priming by the sense primer may be prevented to inhibit the non-specific synthesis of cDNA, thereby increasing specificity and sensitivity of the multiplex RT-PCR.

In addition, in order to increase sensitivity of the kit for inspection of various viruses in a respiratory inspection sample, a method in which primers having homology to rRNA are excluded so that rRNA contained in the respiratory sample does not perform the reverse transcriptase reaction, and oligo in which 3'-terminal corresponding to parts having base sequences complementary to rRNA is blocked, is added together at the time of performing the RT reaction, thereby preventing reverse-transcription and amplification of rRNA, such that RT-PCR may be performed with high sensitivity,

was developed (A sensitive assay for virus discovery in respiratory clinical samples. de Vries M, Deijs M, Canuti M, van Schaik BD, Faria NR, van de Garde MD, Jachimowski LC, Jebbink MF, Jakobs M, Luyf AC, Coenjaerts FE, Claas EC, Molenkamp R, Koekkoek SM, Lammens C, Leus F, Goossens H, Ieven M, Baas F, van der Hoek L.).

Further, on-tube RT-PCR method using real time quantitative PCR in Taqman method provides high sensitivity and high selectivity; however, has a problem in that in a sample containing RNA/DNA such as a sample containing nucleic acid extracted from blood, 100 copy or less of samples may not be detected for each reaction (Espy M. J. et al 2006 Realtime PCR in clinical microbiology:application for routine laboratory testing. Clin. Microbiol. Rev. 19:165-256). In order to solve the problems, a method of primarily performing amplification by 15 cycles of the general PCR, and then performing the real time quantitative PCR thereon has been used; however, it is inconvenient and there is a risk of a false-positive (Highly Sensitive and Broadly Reactive Quantitative Reverse Transcription-PCR Assay for High-Throughput Detection of Rift Valley Fever Virus. Brian H. Bird et al, J Clin Microbiol. 2007 November; 45(11): 35063513. Published online 2007 September 5.).

Similar to the above case, in the case of DNA nucleic acid extract containing DNA fragments being short pieces due to separation • purification processes of bio-samples and decomposition of a sample, the nucleic acid extract is modified at 95°C in the PCR reaction, and then a large number of non-specific nucleic acids are amplified by the non-specific priming in which a number of single stranded DNAs being short pieces function as the primer in the hybridization process, such that the desired target nucleic acid may not be detected.

In order to perform precise inspection of a nucleic acid, it is important to extract the nucleic acid with high efficiency to perform amplification of the nucleic acid with high sensitivity. Even though various kinds of automatic apparatuses for extracting a nucleic acid automatically performing nucleic acid extraction were developed, since a template nucleic acid itself is non-specifically primed and mainly amplified in reactions for amplifying the nucleic acid using nucleic acid polymerase and primer such as the PCR reaction or the real time quantitative PCR reaction for detecting a trace of nucleic acid, a false-positive may occur or it is difficult to detect a target nucleic acid.

Accordingly, the present inventors found a method for preparing a nucleic acid with high sensitivity capable of amplifying and detecting only the target nucleic acid with high sensitivity using a template nucleic acid prepared by previously adding a nucleic acid polymerization terminator terminating the polymerization reaction and polymerase to the template nucleic acid to perform the polymerization termination reaction, and eliminating the nucleic acid polymerization terminator, thereby completing the present invention.

More specifically, it was found from the method for preparing the nucleic acid with high sensitivity that the nucleic acid polymerization terminator is added to the template nucleic acid used in detecting the nucleic acid using the nucleic acid polymerization such as the PCR reaction or the real time quantitative PCR reaction for detecting a trace of nucleic acid, such that the non-specific self-priming inhibiting the amplification reaction of the target nucleic acid and the subsequent non-specific amplification may be basically eliminated.

[References]

[patent publications]

KR 10-1098764 B1, laid open on December 20, 2011

[Non-patent publication]

(publication 1) Malgoyre A et al., Biochem Biophys Res Commun. 2007 Mar 2; 354(1) :246-52. Epub 2007 Jan 3.
(publication 2) Espy M. J. et al., Clin. Microbiol. Rev. 19:165-256, 2006)
(publication 3) Brian H. Bird et al, J Clin Microbiol. 2007 November; 45(11): 35063513. Published online 2007 September 5.

**[Disclosure]**

[Summary of Invention]

An object of the present invention is to provide a method for treating a nucleic acid before amplifying the nucleic acid using a nucleic acid polymerization reaction to prevent a non-specific priming of the nucleic acid. In order to achieve the foregoing objects, the present invention provides a method for treating a nucleic acid before amplifying the nucleic acid using a nucleic acid polymerization reaction to prevent a non-specific priming of the nucleic acid, the method comprising: (1) terminating 3'-terminal of the nucleic acid by reacting a reaction mixture comprising nucleic acid, nucleic

acid polymerase, an enzyme reaction buffer, and a nucleic acid polymerization terminator inhibiting an elongation of 3' terminal of the nucleic acid; and (2) inactivating or eliminating non-reacted free nucleic acid polymerization terminator from the reaction mixture.

[Description of Drawings]

FIG. 1 is a result of Comparative Example 1 of the present invention showing an electrophoresis photograph of a reverse transcriptase-polymerase chain reaction product by short RNA fragments (A: RNA oligo nucleotide, B: DNA oligo nucleotide).

FIG. 2 is a result of Comparative Example 2 of the present invention showing an electrophoresis photograph of non-specific reverse transcriptase polymerase chain reaction and PCR reaction products by short RNA fragments.

FIG. 3 is a result of Example 1 of the present invention showing termination by reaction of 3'-deoxyadenosine 5'-triphosphate (3'-dATP) using Maldi-tof-mass spectrometer.

FIG. 4 is a result of Example 1 of the present invention showing an electrophoresis photograph of a non-specific reverse transcriptase polymerase chain reaction product of a single-stranded RNA terminated by 3'-deoxyadenosine 5'-triphosphate (3'-dATP) (A: RNA oligo nucleotide not having synthesized 3'-deoxyadenosine 5'-triphosphate (3'-dATP), B: RNA oligo nucleotide having synthesized 3'-deoxyadenosine 5'-triphosphate (3'-dATP)).

FIG. 5 is a result of Comparative Example 3 of the present invention showing confirmation of a real-time reverse transcriptase polymerase chain reaction inhibition phenomenon by RNA extracted from human serum (A: a sample extracted from DEPC-D.W, B: a sample extracted from human serum).

FIG. 6 is a result of Example 2 of the present invention showing confirmation of a real-time polymerase chain reaction inhibition phenomenon by termination of RNA present in human serum (A: a template RNA, B: RNA generally extracted from human serum was added to a template RNA, C: RNA generally extracted from human serum was terminated and then added to a template RNA).

FIG. 7 is a result of Example 2 of the present invention showing confirmation of activation inhibition of 3'-deoxyadenosine 5'-triphosphate (3'-dATP) using alkaline phosphatase (CIP) (A: a sample obtained by terminating RNA and hydrolyzing 3'-deoxyadenosine 5'-triphosphate (3'-dATP) using CIP, B: a sample containing 3'-deoxyadenosine 5'-triphosphate (3'-dATP) without purification after RNA was terminated by 3'-deoxyadenosine 5'-triphosphate (3'-dATP), C: a sample from which 3'-deoxyadenosine 5'-triphosphate (3'-dATP) is eliminated by AccuPrep PCR Purification).

FIG. 8 is a result of Example 3 of the present invention showing confirmation of a non-specific reverse transcriptase polymerase chain reaction inhibition effect by RNA self-priming through RNA termination using general reverse transcriptase polymerase chain reactions (A and C) and hot-start reverse transcriptase polymerase chain reaction (B and D) (A: RNA which was not terminated, B: RNA which was not terminated, C: terminated RNA, D: terminated RNA) .

FIG. 9 shows a buffer cartridge according to the present invention.

FIG. 10 is an experimental flow chart showing a nucleic acid preparation method using the buffer cartridge of FIG. 9.

FIG. 11 is a diagram showing non-specific reaction inhibition effect by blocking oligonucleotide.

FIG. 12 shows confirmation of non-specific reaction inhibition effect depending on various kinds of modifications used at the time of constructing a blocking oligonucleotide, wherein A is a positive control group using a product having a hot start function, B to G show cases modified by amine, phosphate, C3-space, C6-space, C12-space, and C18-space, lanes 1 to 3 show results obtained by amplifying three different target bases, respectively, and lane M shows a 100 bp DNA ladder capable of differentiating a DNA size.

FIG. 13 with A, which shows a primer dimer and a non-specific reaction inhibition effect depending on addition of the blocking oligonucleotide in a real-time PCR reaction, wherein a horizontal axis indicates a PCR reaction cycle; a vertical axis indicates a fluorescence value measured depending on the reaction cycle; a line I shows a result of a fluorescence detection curve of a control group; and a line II shows a result of a fluorescence detection curve of an experimental group, and B is a graph showing a melting curve using a fluorescence measurement curve, wherein a horizontal axis indicates a temperature change; a vertical axis indicates a fluorescence value measured depending on the temperature increase; the line I shows a result of a melting curve of a control group; and the line II shows a result of a melting curve of an experimental group.

[Best Mode]

Hereinafter, the present invention will be described in detail.

In order to perform precise inspection for a nucleic acid, it is important to not only extract a nucleic acid with

high efficiency but also to amplify and detect the extracted nucleic acid with high sensitivity in a reaction for amplifying the nucleic acid. Even though various kinds of automatic apparatuses for extracting a nucleic acid automatically performing nucleic acid extraction were developed, when detecting a trace of nucleic acid in a reaction for amplifying the nucleic acid using nucleic acid polymerase and primer such as a PCR reaction or a real time quantitative PCR reaction, a false-positive may occur or it is difficult to detect a target nucleic acid.

Accordingly, the present inventors found that a problem occurs since the nucleic acid itself is non-specifically self-primed and mainly amplified in inspections for amplifying the nucleic acid such as the PCR reaction or the real time quantitative PCR reaction for detecting a target nucleic acid, and thus, a material terminating a polymerization reaction and nucleic acid polymerase are added to the nucleic acid, followed by termination, such that the nucleic acid is not capable of functioning as a primer, whereby only the target nucleic acid may be amplified and detected with high sensitivity, thereby completing the present invention.

In one aspect, the present invention provides a method for treating a nucleic acid before amplifying the nucleic acid using a nucleic acid polymerization reaction to prevent a non-specific priming of the nucleic acid, the method comprising:

1) terminating 3'-terminal of the nucleic acid by reacting a reaction mixture comprising nucleic acid, nucleic acid polymerase, an enzyme reaction buffer, and a nucleic acid polymerization terminator inhibiting an elongation of 3' terminal of the nucleic acid; and
2) inactivating or eliminating the nucleic acid polymerization terminator from the reaction mixture.

More specifically, the present invention provides a method for treating a nucleic acid used in amplifying and detecting a nucleic acid by a nucleic acid polymerization reaction, the method comprising:

1) separating and purifying the nucleic acid from a biological sample containing the nucleic acid;
2) terminating 3'-terminal of the separated and purified nucleic acid of step 1) by using a reaction mixture containing nucleic acid polymerase, an enzyme reaction buffer, and a nucleic acid polymerization terminator inhibiting an elongation of 3' terminal ; and
3) inactivating or eliminating the nucleic acid polymerization terminator from the reaction mixture containing the nucleic acid having the terminated 3'-terminal.

The method for treating the nucleic acid according to the present invention may be applied to generally known automatic apparatuses for extracting a nucleic acid, for example, the apparatuses disclosed in Korean Patent No. 148239, US Patent No. 5702590, US Patent No. 5647994, European Patent No. 0691541, US Patent No. 5336760, US Patent No. 5897783, US Patent No. 6187270, and Korean Patent Application No. 10-2009-025743. In addition, ExiPrep™ 16-fully Automated DNA/RNA/Proteins Purification System manufactured by Bioneer corporation may be preferably used as the automatic apparatus for extracting the nucleic acid of the present invention.

In the present invention, a sample may be samples derived from any plants, animals, bacteria, or viruses containing the nucleic acid which is polynucleotide.

In the present invention, the nucleic acid is RNA or DNA, and the nucleic acid polymerase includes all nucleic acid polymerases capable of attaching a terminator to 3'-terminal of DNA or RNA so that polymerization does not progress any further.

More specifically, it is preferable that the nucleic acid polymerase of the present invention does not have an 3' $\rightarrow$ 5' exonuclease activity, such that the terminator is not hydrolyzed again, and more preferably, titer of enzyme might be lost at a high temperature over 90°C. The nucleic acid polymerase may include at least one selected from the group consisting of RNA-dependent RNA-polymerase, RNA-dependent DNA-polymerase, DNA-polymerase, and RNA-polymerase.

The RNA may be mRNA, RNA without poly-A tail, or RNA of virus or prokaryote, but the present invention is not limited thereto.

In the present invention, the used RNA-dependent RNA-polymerase is a general enzyme which is mainly contained in the virus and synthesizes RNA having RNA as a template, and NS5 enzyme derived from Flavivirus such as dengue virus or foot and mouth disease virus may be used.

In addition, the DNA-polymerase used in the present invention indicates an enzyme synthesizing a new DNA strand by using a DNA strand as a template. The DNA-polymerase includes Pol I-type DNA-polymerase (for example: Escherichia coli DNA-polymerase I, Klenow fragment and Taq DNA-polymerase), non-$\alpha$-, non-PolI-type DNA-polymerase (for example: DNA-polymerase disclosed in WO 97/24444), but the present invention is not limited thereto.

The RNA-dependent DNA-polymerase is a reverse transcriptase enzyme, and various reverse transcriptase enzymes such as MMLV reverse transcriptase, AMV reverse transcriptase, HIV reverse transcriptase, and the like, may be used. In addition, a terminal deoxynucleotidyl transferase such as poly(A) polymerase attaching adenosine to 3'-terminal may be included as a RNA-polymerase.

For example, as a HIV reverse transcriptase enzyme, 3'-azido-3'-deoxythymidine 5'-triphosphate which is an activated form of the reverse transcriptase reaction in human body of AZT (3'-azido-3'-deoxythymidine) used as an HIV therapeutic agent showing reactivity 100-times higher than that of human-derived DNA-polymerase may be effectively used in the present invention (Phosphorylation of 3'-azido-3'-deoxythymidine and selective interaction of the 5'-triphosphate with human immunodeficiency virus reverse transcriptase, P A Furman, J A Fyfe, M H St Clair, K Weinhold, J L Rideout, G A Freeman, S N Lehrman, D P Bolognesi, S Broder, and H Mitsuya, PNAS November 1, 1986 vol. 83 no. 21 8333-8337).

The term "nucleic acid polymerization terminator" used herein means a material terminating a polymerization reaction of all nucleic acid fragments so that the nucleic acid is not elongated any further, and a material bound to a terminal of the nucleic acid fragment so that nucleic acid chain elongation does not further occur.

The term "termination" used herein means that the nucleic acid polymerization reaction does not progress any further by covalently binding "the nucleic acid polymerization terminator" to 3'-terminal of the nucleic acid, wherein the used material is defined as a terminator.

More specifically, the term "termination" mentioned herein means that the nucleic acid polymerization reaction does not progress any further by covalently binding a material without hydroxyl groups at 3' position of the nucleic acid, or a material having chemical groups in which the polymerization reaction by the polymerase does not progress, to 3'-terminal of the nucleic acid, wherein the used material is defined as a terminator.

In the present invention, the nucleic acid polymerization terminator is a nucleic acid-like compound which is activated as a triphosphate form capable of functioning on the nucleic acid polymerase, and various kinds of nucleotide triphosphate in which 3' hydroxyl group to be connected to the nucleic acid is not present, or is substituted with other groups, may be used, and the nucleic acid polymerization terminator having good reactivity with each nucleic acid polymerase may be selected to be used.

In the present invention, the nucleic acid-like compound may be at least one selected from the group consisting of 2'3'-dideoxynucleoside 5'-triphosphate, 3'-deoxyadenosine 5'-triphosphate, 3'-azido-3'-deoxythymidine 5'-triphosphate, 1-β-d-arabinofuranosylnucleoside 5'-triphosphate, acyclo-guanosine triphosphate, 3'-amino-3'-deoxynucleoside 5'-triphosphate, and 3'-fluoro-3'-deoxynucleoside 5'-triphosphate.

More specifically, as the DNA-polymerase derived from Escherichia coli or thermoduric bacteria, dideoxy nucleotide triphosphate (2'3'-dideoxy nucleotide triphosphate: ddNTP) largely used in dideoxy base sequence determination developed by Sanger may be used.

The term "dideoxy nucleotide triphosphate (ddNTP)" used herein may include at least one selected from the group consisting of dideoxy guanosine triphosphate (ddGTP), dideoxy adenosine triphosphate (ddATP), dideoxy thymidine triphosphate (ddTTP) and dideoxy cytidine triphosphate (ddCTP).

The deoxy nucleotide triphosphate (dNTP) may include at least one selected from guanosine, adenosine, thymidine, uridine, and cytidine.

As an example thereof, in order to perform the termination reaction by using the DNA-polymerase, various nucleic acid polymerization terminators may be used. In the case of using mammal-derived DNA-polymerase, 5'-Triphosphate 1-β-d-Arabinofuranosylcytosine or 5'-Triphosphate 9-β-d-Arabinofuranosyladenine having high reactivity to the enzyme may be used (Inhibition of Mammalian DNA Polymerase by the 5'-Triphosphate of 1-β-d-Arabinofuranosylcytosine and the 5'-Triphosphate of 9-β-d-Arabinofuranosyladenine, J. J. Furth, and Seymour S. Cohen, Cancer Res October 1968 28; 2061).

In addition, triphosphate of acyclovir nucleic acid (acyclonucleoside) which has largely been used as an antiviral agent inhibiting the virus-derived DNA-polymerases may also be used as a nucleic acid polymerization terminator (Inhibition of herpes simplex virus-induced DNA polymerase activity and viral DNA replication by 9-(2-hydroxyethoxymethyl)guanine and its triphosphate. P A Furman, M H St Clair, J A Fyfe, J L Rideout, P M Keller and G B Elion, J. Virol. October 1979 vol. 32 no. 1 72-77).

As another example thereof, Poly A polymerase which is RNA polymerase, which is an enzyme attaching adenosine to 3'-terminal of RNA, is an enzyme suitable for the nucleic acid polymerization termination reaction of the present invention. When using the enzyme, 3'-deoxyadenosine triphosphate (3'-dATP, cordycepin) which is known as an inhibitor of the poly A polymerase, may be used.

In the present invention, step 2) is a process of inactivating or eliminating unreacted nucleic acid polymerization terminator after step 1), wherein in the inactivating of the nucleic acid polymerization terminator, all various enzymes capable of hydrolyzing the triphosphate bond of the various kinds of nucleic acid polymerization terminator used in the polymerization may be used.

More specifically, in the inactivating of the nucleic acid polymerization terminator, the nucleic acid polymerization terminators are hydrolyzed to be inactivated, and function of the enzyme needs to be lost, such that it is preferable to easily lose the activity by heat. The reason is that nucleotide triphosphate in the subsequent reverse transcriptase reaction or the PCR reaction should not be decomposed.

For example, the phospho-bound hydrolase is alkaline phosphatase having a substrate property decomposing triphosphate, and bacterial alkaline phosphatase (BAP) and calf intestinal phosphatase (CIP), and the like, may be used, and more preferably, CIP which is easily inactivated by heat may be used.

In addition, alkaline phosphatases derived from various organisms hydrolyzing pyrophosphate, ATP, and the like, that is, Escherichia coli, calf small intestine, and shrimp, and Autotaxin may also be used (Clair T, Lee HY, Liotta LA, Stracke ML (1997). "Autotaxin is an exoenzyme possessing 5'-nucleotide phosphodiesterase/ATP pyrophosphatase and ATPase activities". J. Biol. Chem. 272 (2): 996~1001. doi:10.1074/jbc.272.2.996. PMID 8995394.). *Staphylococcus aureus* adenosine synthase (AdsA) which non-specifically decomposes nucleotide triphosphate may be used.

The method for treating the nucleic acid according to the present invention might comprise performing a process for separating and purifying a nucleic acid, and subsequently, the nucleic acid polymerase and the nucleic acid polymerization terminator are added to the extracted nucleic acid, to thereby terminate the polymerization reaction of the complementary nucleic acid, thereby eliminating the non-specific priming by the nucleic acid. After the reaction, since the unreacted nucleic acid polymerization terminators function as an inhibitor of the subsequent polymerization chain reaction or the PCR reaction, the unreacted nucleic acid polymerization terminator is required to be eliminated.

Gel filtration, or chaotropic agent and silicarbide may be used to eliminate the nucleic acid polymerization terminator, but the present invention is not limited thereto.

In the method for treating the nucleic acid according to the present invention, known integrated apparatuses for analyzing a nucleic acid which integrally manages known automatic apparatus for extracting a nucleic acid and a gene amplification apparatus may be used, and preferably, Exiprep 16 DX (manufactured by Bioneer corporation) may be used, but the present invention is not limited thereto.

More specifically, the process for separating and purifying the nucleic acid is performed, and subsequently, the nucleic acid polymerase and the nucleic acid polymerization terminator are added to the extracted nucleic acid, to thereby perform the nucleic acid polymerization termination reaction, thereby inactivating the priming function of all nucleic acids. Since the terminators remaining after the reaction function as an inhibitor of the subsequent reverse transcriptase reaction or the PCR reaction, two methods may be used to eliminate the remaining terminators.

One of the two methods is to perform RNA purification again by using Exiprep 16 DX (manufactured by Bioneer corporation) and the other one is to decompose the nucleic acid polymerization terminator so that the reaction is not performed any further.

The method of performing the purification by using the apparatus, which is a method of attaching silica magnetic particles in the presence of chaotropic agent, followed by washing and elution processes, is performed by various steps, such that there are problems in that a plenty of time is required, and the purified RNA may be lost in various steps. Meanwhile, the method of decomposing the nucleotide triphosphate which is an activated nucleic acid polymerization terminator into di- or mono-trinucleotide by a hydrolase to be inactivated is rapid and easy, and does not deteriorate yield.

The inactivation method using the enzyme will be described in detail. Since a temperature of a well used in the elution step collecting the magnetic particles of Exiprep 16 DX is controllable, the well is eluted with an elution buffer, then alkaline phosphatase which is triphosphate hydrolase, autotaxin, *Staphylococcus aureus* adenosine synthase (AdsA) are added thereto, and is reacted at 37°C, such that the terminator may be easily eliminated.

As a specific example, the method of performing the purification by using Exiprep 16 DX (manufactured by Bioneer corporation) may use a buffer cartridge. The buffer cartridge of the present invention may consist of a total of 12 wells and one sample tube. Wells ① to ⑩ are to extract and purify the nucleic acid, and the constitution thereof is as follows:

> Well ① is a well in which a reagent is mixed and reaction is performed, Well ② is a well containing an enzyme required for extracting the nucleic acid, Well ③ is a well containing a cell lysis solution performing cell lysis of blood, blood serum, and culture cells, Well ④ is a well containing a binding solution for binding the eluted nucleic acid to a silica bead, Well ⑤ is a well containing silica beads for binding the nucleic acid, and Well ⑥ is a well containing a primary washing solution for eliminating pollutants remaining in the silica beads having the nucleic acids bound thereto. Well ⑦ is a well containing a secondary washing solution for eliminating pollutants remaining in the silica beads having the nucleic acid bound thereto, Well ⑧ is a well containing a tertiary washing solution for eliminating pollutants remaining in the silica beads having the nucleic acid bound thereto, Well ⑨ is a well containing a tip washing solution for clearly washing a tip used in the extraction of the nucleic acid, and Well ⑩ is a well containing an elution solution for eluting the nucleic acid from the silica beads having the nucleic acid bound thereto. FIG. 9 is referred.

More specifically, the sample contained in the sample tube is transferred to Well ①, and the cell lysis solution contained in Well ③ is taken and mixed with enzymes required for extracting the nucleic acid contained in well ②, and then transferred to Well ① containing the sample. All cells were decomposed by raising a temperature to be 60°C, and all solutions of Well ① is transferred to Well ④ containing the nucleic acid binding solution and mixed together. All

reaction solutions are taken and transferred to Well ⑤ containing the silica beads, and all of the nucleic acids exposed in the reaction solution are bound to the silica beads. Remaining reaction solution is completely discarded while leaving only the silica beads having the nucleic acid bound thereto in Well ①. Surface of the silica beads are sequentially washed with the primary washing solution contained in Well ⑥, the secondary washing solution contained in Well ⑦, and the tertiary washing solution contained in Well ⑧, such that purified nucleic acids only are left on the surface of the silica. For final extraction of the nucleic acid, a surface of the tip used in extracting the nucleic acid is washed by using a tip washing solution, then the nucleic acid elution solution is taken from Well ⑩ containing the nucleic acid elution solution, and mixed with the silica beads having the nucleic acids, thereby separating the nucleic acid from the silica beads, and eluting the nucleic acid with the nucleic acid elution solution.

All of poly (A) polymerase and 3'-dATP, the reaction solution contained in Well ⑪ are taken and transferred to the eluted nucleic acid solution, followed by reaction at 37°C for 10 minutes, thereby completing the termination reaction at 3'-terminal of the extracted ribonucleic acid. Here, since 3'-dATP used in the present reaction inhibits the reverse transcriptase reaction or the PCR reaction, 3'-dATP should be necessarily removed. In order to solve the problem, the following two methods may be used.

When explaining with reference to FIG. 10, whole processes are firstly performed according to RNA extraction and purification, and RNA termination, and in order to eliminate the activity of 3'-dATP used in the RNA termination, the following two methods, that is, elimination of 3'-dATP through RNA purification, or hydrolysis of triphosphate of 3'-dATP using phosphatase, are then performed.

The first method is a purification method using reagents used in extracting the nucleic acid, which is 'a process for eliminating 3'-dATP through the RNA purification'. All of the reaction solution and the silica beads obtained after performing the RNA termination reaction are taken and transferred to Well ④ containing the nucleic acid binding solution used in the extraction of the nucleic acids. Remaining reaction solution is completely discarded while leaving only the silica beads mixed with the nucleic acid binding solution to be bound to the nucleic acids in Well ①. The surface of the silica beads is washed by using the tertiary washing solution contained in Well ⑧, such that purified nucleic acids only are left on the surface of the silica. Then, the nucleic acid elution solution is taken from Well ⑩ containing the nucleic acid elution solution, and mixed with the silica beads having the nucleic acids, thereby separating the nucleic acid from the silica beads, and eluting the nucleic acid with the nucleic acid elution solution.

The second method is to add alkaline phosphatase to the reaction solution obtained after performing the RNA termination reaction to eliminate triphosphate residue present in 5'-terminal of 3'-dATP, thereby preventing occurrence of side reactions in the subsequent PCR process, which is 'a process for hydrolyzing 3'-dATP using alkaline phosphatase'. All of the reaction solution obtained after performing the RNA termination reaction are taken and transferred to Well ⑫ containing the alkaline phosphatase and well-mixed together. The mixed reaction solution is taken and transferred to Well ①, followed by reaction at 37°C for 10 minutes, thereby eliminating all of the phosphate residue at 5'-terminal of ddATP. In order to inactivate alkaline phosphatase used in the reaction, a temperature is increased to 65°C and the reaction is performed for 10 minutes. After the reaction is completed, all of the nucleic acids eluted while leaving only the silica beads only are taken to be used for the PCR reaction.

In another aspect, the present invention also provides a method for polymerizing a nucleic acid using a nucleic acid for nucleic acid polymerization reaction in which a nucleic acid polymerization terminator is bound to 3'-terminal to prevent the non-specific nucleic acid polymerization.

In the present invention, the amplification method may be used for hot-start PCR, nested PCR, multiplex PCR, reverse transcriptase PCR (RT-PCR), real time PCR, degenerate oligonucleotide primer (DOP) PCR, quantitative RT-PCR, and the like, as well as general PCR reactions. In addition, the amplification method may also be applied to all methods amplifying signals of specific base sequences by the selectivity of the primer and the polymerization reaction of the nucleic acid polymerase, thereby being used for various methods for inspecting the amplification of polymerases, such as isothermal rolling circle amplification, isothermal amplification using transcription, and the like.

Also described is a kit for preparing a nucleic acid, including a nucleic acid polymerase, an enzyme reaction buffer, and a nucleic acid polymerization terminator inhibiting elongation of 3'-terminal of the nucleic acid required for the method for preparing a nucleic acid used in amplifying and detecting the nucleic acid using the nucleic acid polymerization reaction.

The kit for preparing the nucleic acid as described herein may include at least one selected from the group consisting of RNA-dependent RNA-polymerase, RNA-dependent DNA-polymerase, DNA-polymerase, and RNA-polymerase, as the nucleic acid polymerase, and preferably, may include poly(A) polymerase.

The kit for preparing the nucleic acid is described as including Tris-HCl, KCl, $(NH_4)_2SO_4$, $MgSO_4$, and Triton X-100, as the enzyme reaction buffer.

It is further described that the kit for preparing the nucleic acid may include at least one selected from the group consisting of 2'3'-dideoxynucleoside 5'-triphosphate, 3'-deoxyadenosine 5'-triphosphate, 3'-azido-3'-deoxythymidine 5'-triphosphate, 1-β-d-arabinofuranosylnucleoside 5'-triphosphate, acyclo-guanosine triphosphate, 3'-amino-3'-deoxynucleoside 5'-triphosphate, and 3'-fluoro-3'-deoxynucleoside 5'-triphosphate, as the nucleic acid polymerization terminator.

The kit for preparing the nucleic acid as described herein may further include a phospho-bound hydrolase hydrolyzing triphosphate which inactivates the nucleic acid polymerization terminator, wherein as the phospho-bound hydrolase, at least one enzyme selected from the group consisting of alkaline phosphatases derived from Escherichia coli, calf small intestine, and shrimp, and autotaxin, may also be used.

The kit as described herein may include a guidebook. The "guidebook" is a printed matter describing the usage of the kit, for example, a method for preparing a reagent solution for preparing a nucleic acid, preparation conditions to be present, and the like. The guidebook includes description on a surface of a leaflet such as pamphlet or brochure, a label attached to the kit, and a package including the kit. In addition, the guidebook includes information published or provided through electrical media such as the Internet.

Also described is an automatic apparatus for purifying a nucleic acid, including a storage part storing the nucleic acid polymerase and the nucleic acid polymerization terminator; and a supply part supplying the nucleic acid polymerase and the nucleic acid polymerization terminator to a sample containing a target nucleic acid.

The storage part as described may further contain triphosphate hydrolase, and an enzyme reaction buffer, and the apparatus may further include a computer controller to achieve whole automation.

Further described herein is fully automatic equipment for inspecting a nucleic acid, including the automatic apparatus for purifying a nucleic acid.

As the fully automatic equipment for inspecting a nucleic acid, known integrated apparatuses for analyzing a nucleic acid which integrally manage known automatic apparatus for extracting a nucleic acid and a gene amplification apparatus may be used. A fully automatic equipment for analyzing a nucleic acid which fully automatically purifies, amplifies and analyzes the nucleic acid developed by the present applicants (Korean patent application Nos. 10-2010-0086468 and 10-2012-0013757) may be used. As the real time gene amplification apparatus, known real time gene amplification apparatuses may be used. Preferably, an apparatus disclosed in Korean Patent No. 10-794703, an apparatus disclosed in PCT/KR2008/064558, or Exicycler 96 Real-Time Quantitative Thermal Block manufactured by Bioneer Corp., may be used..

Further described is a composition for polymerizing a nucleic acid containing the nucleic acid in which the nucleic acid polymerization terminator is bound to 3'-terminal to prevent non-specific nucleic acid polymerization, $Mg^{2+}$ ion, 4 kinds of dNTPs, DNA-polymerase, and a primer for amplifying the nucleic acid.

The composition for polymerizing the nucleic acid as described herein may further contain a primer, a probe, a fluorescent dyes, a reverse transcriptase enzyme, and the like, as needed. The term "nucleic acid" used herein includes a template nucleic acid to be amplified, DNA, RNA, a hybrid of DNA and RNA, a mixture thereof, a nucleic acid extract or a nucleic acid sample, and the like, without limitation.

The composition for polymerizing the nucleic acid as described herein may further contain a blocking oligonucleotide having complementary base sequence to the primer for amplifying the template nucleic acid and a blocked 3'-hydroxyl group.

The number of base sequences of the blocking oligonucleotide is smaller than that of the primer having complementary base sequence. When the number of base sequences of the blocking oligonucleotide is smaller than that of the primer as described above, a melting temperature of a primer (or a template nucleic acid)-blocking oligonucleotide bond is lower than that of a primer-template target DNA bond. Therefore, the primer and the blocking oligonucleotide are bound by a double helix structure at a low temperature; however, the blocking oligonucleotide having slightly low denaturalization temperature is dissociated before reaching an annealing temperature of the primer, such that the double helix structure is not formed any further, which does not have an effect on the PCR reaction, thereby being effectively used for the hot start PCR. The method of using the blocking oligonucleotide as described above may be applied to the probe in the same manner. The blocking oligonucleotide may have a melting temperature lower than that of the primer by 1°C or more, e.g. 2°C or more. The blocking oligonucleotide may have a melting temperature of at least 25°C or more, e.g. 50°C or more.

In addition, the blocking oligonucleotide lacks a hydroxyl group at 3'-terminal or has other substituent groups rather than the hydroxyl group at 3'-terminal. The DNA polymerization method progresses by a manner in which the hydroxyl group at 3'-terminal and phosphate group at 5'-terminal are bound to each other, such that when the hydroxyl group at 3'-terminal is substituted with the other different substituent groups, the DNA polymerization reaction does not progress any further. Also described are fluorescent materials (dye), quencher, or materials represented by the following Chemical Formulas shown in Table 1 below as the substituent group..

[Table 1]

| Substituent Group | Chemical Formulas |
|---|---|
| Hydrogen | -H |

(continued)

| Substituent Group | Chemical Formulas |
|---|---|
| C6 amine | 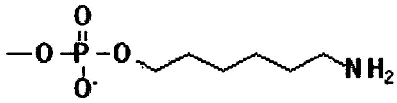 |
| phosphate | 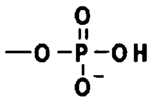 |
| Biotin | 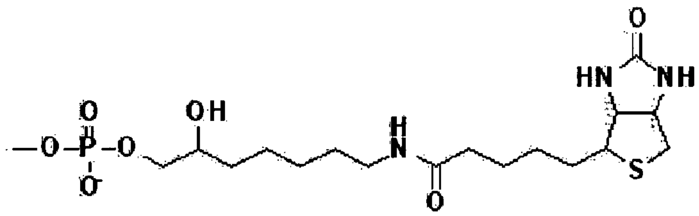 |
| C3 spacer | 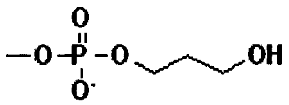 |
| C6 spacer | 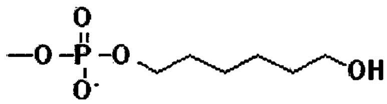 |
| C12 spacer | 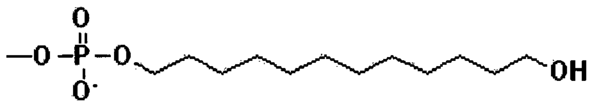 |
| C18 atom spacer | 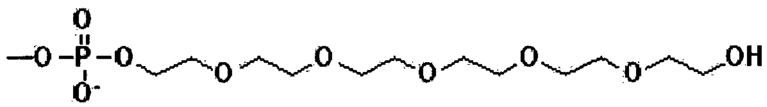 |
| Thiol | 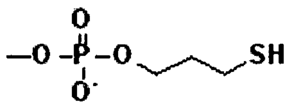 |
| Inverted dN | 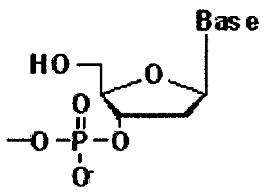 |
| DNP (2,4-dinitrophenyl) -TEG | 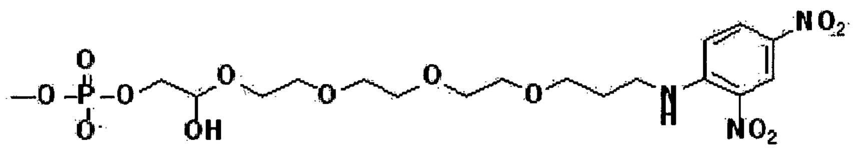 |
| Maleimide | 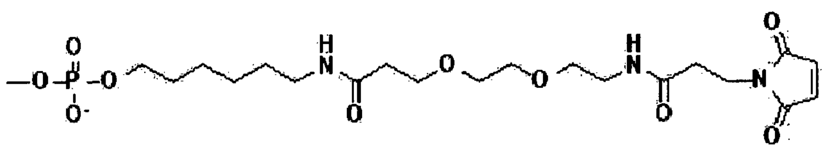 |

(continued)

| Substituent Group | Chemical Formulas |
|---|---|
| Dithiol | 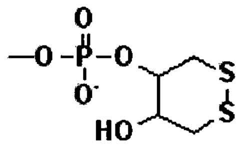 |
| 3'AMCA (aminomethyl-coumann-acetate) | 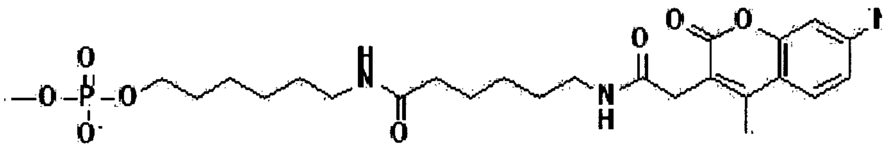 |
| 3' Amide | 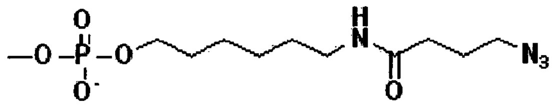 |
| 3' acrylamide (acrydite) | 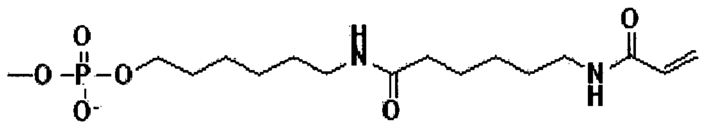 |

It is further described that the first base at 5'-terminal of the blocking oligonucleotide forms a complementary binding to the first base at 3'-terminal of the primer complementarily bound to the blocking oligonucleotide, or forms a complementary binding to the second or the following bases at 3'-terminal of the primer, thereby preventing at least one base at 5'-terminal of the blocking oligonucleotide from being unpaired. That is, it is preferable that all bases at 5'-terminal of the blocking oligonucleotide form the complementary binding to the bases at 3'-terminal of the primer. If at least one base at 5'-terminal of the blocking oligonucleotide is unpaired, the DNA polymerization reaction initiates from 3'-terminal of the primer to the first base at 5'-terminal of the blocking oligonucleotide, and as a result, a length of the primer may be further elongated. In the case in which the length-elongated primer as described above is present in the reaction solution, non-specific amplification resulting products by formation of the primer-dimer which is the existing problem, may be more increased, which is not preferred.

In addition, the blocking oligonucleotide may be specifically bound to any one of a forward primer and a reverse primer, or all of the forward primer and the reverse primer, with respect to the primer couple having complementary sequence to the blocking oligonucleotide.

In the present invention, the buffer for a reaction is preferably 10 mM TrisHCl, 40 mM KCl, and a pH of 9.0. Four kinds of the dNTPs are dATP, dTTP, dGTP and dCTP. As the DNA-polymerase, any known DNA-polymerases may be used without limitation. Among them, a polymerase having 5'-> 3' exonuclease activity, a polymerase having 3'-> 5' exonuclease activity, and a polymerase without 5'-> 3' exonuclease activity and 3'-> 5' exonuclease activity may be used alone, or may be used in combination with the DNA-polymerases. An example of the polymerase having 5'-> 3' exonuclease activity includes a Taq DNA-polymerase, an example of the polymerase having 3'-> 5' exonuclease activity includes a Pfu DNA-polymerase or a TLA DNA-polymerase, and an example of the polymerase without 5'-> 3' exonuclease activity and 3'-> 5' exonuclease activity includes a Top DNA-polymerase, but the present invention is not limited thereto. The DNA-polymerase may be contained in the PCR composition with a concentration of 0.1 to 10 U(unit), preferably, 0.5 to 2 U, most preferably, 1 U.

Also described is a composition for polymerizing the nucleic acid of the present invention which may further contain dye materials which are non-reactive to a nucleic acid to provide convenience for the experiment, prevent pollution by the PCR reaction product, stabilize the DNA-polymerase and dNTP, and improve reactivity. The non-reactive dye material may be selected from materials not having an effect on the PCR reaction, and examples of the materials satisfying this condition include aqueous dyes such as bromophenol blue, xylene cyanole, bromocresol red, cresol red, and the like. The non-reactive dye materials may have a content of 0.0001 to 0.01 wt% based on the total composition, and the content is preferably 0.001 to 0.005 wt%, more preferably, 0.001 to 0.003 wt%. If the non-reactive dye material is added in a content of less than 0.0001 wt%, since the dye has a low concentration at the time of performing agarose gel electrophoresis for analysis after the PCR reaction, it is difficult to observe the transfer of the sample with the naked eye, and if the non-reactive dye material is added in a content of more than 0.01 wt%, at the time of performing the PCR reaction, high concentrated aqueous dye may function as a reaction inhibitor. In addition, after precipitation in the agarose

gel, the specimen may be prevented from being transferred at the time of performing electrophoresis.

Further, the composition for polymerizing the nucleic acid as described herein may contain a reverse transcriptase enzyme for synthesizing cDNA if needed. As described above, in a case of the reverse transcriptase PCR containing the reverse transcriptase enzyme, the blocking oligonucleotide with respect to a sense primer of RNA to be amplified is preferably added to the composition for the hot start PCR of the present invention.

The DNA-polymerase is an enzyme generating a polymerization reaction by binding 5'-terminal to 3'-terminal of the partial double helix structure composed of single line. In the present invention, a blocking oligo was designed by considering properties of the DNA-polymerase. In the present invention, it was designed so that 5'-terminal of the blocking oligo is complementarily bound to 3'-terminal of the primer, thereby preventing the primer from being non-specifically reacted any further, and preventing the primer from being hybridized to other nucleic acids to elongate the bases. In addition, 3'-terminal of the blocking oligo has a length shorter than that of 5'-terminal of the primer, such that the DNA-polymerase is well attached, and simultaneously, in order to prevent the blocking oligo from being elongated, the 3' hydroxyl group was blocked with other substituent groups. Accordingly, just by the addition of the blocking oligonucleotide to the reaction product, the DNA-polymerase is attached to the double helix of the primer and the blocking oligo, such that the non-specific reaction may not be performed any further, thereby preventing the expansion reaction of the primer at room temperature.

In addition, the blocking oligonucleotide used for the composition for hot start PCR of the present invention, which is an oligonucleotide having complementary base sequence to the primer or the probe, has a length shorter than that of the primer/the probe to have a double helix structure with the primer/the probe at a low temperature, and they are bound to each other as the DNA-polymerase recognizes the double helix structure as a substrate; however, since 3'-terminal is blocked, the DNA polymerization does not occur. Therefore, the non-specific polymerization of the primer which may be a problem in the general PCR reaction may be inhibited to prevent the occurrence of non-specific PCR product. Therefore, when using the composition for hot start PCR as described herein, unlike hot start PCR methods using antibody, chemical formula and pyrophosphate, used in the related art, the polymerase is directly detached below the melting temperature of the primer-target template DNA double helix, to be activated, such that the PCR reaction may be directly performed without consuming incubation time for activation.

When considering an action mechanism of the blocking oligonucleotide, the forward primer and the reverse primer function as an essential factor in the PCR reaction. The blocking oligonucleotide is an oligonucleotide consisting of complementary base sequences having a nucleotide, wherein the number of nucleotides is smaller by one or more than that of the forward primer or the reverse primer, which has high specificity to the primer. Therefore, when mixing the forward/reverse primer with the blocking oligonucleotide, the blocking oligonucleotide is bound to the forward/reverse primer to prevent the forward/reverse primer from functioning, thereby previously preventing the primer-dimer from being formed and the participation to the non-specific reaction. When reaching an appropriate temperature, that is, the melting temperature of the blocking oligomer, the binding between the primer and the blocking oligonucleotide is broken, such that the primer is bound to the target template nucleic acid, thereby precisely and effectively achieving the PCR reaction. That is, since the blocking nucleotide is constructed to have a relatively short length as compared to the primer/the probe, when reaching the appropriate temperature at which the primer is bound to the template nucleic acid, the blocking nucleotide is dissociated from the primer/the probe, which does not have an effect on the binding of the primer to the template.

In the present invention, the blocking oligonucleotide is a blocking oligo RNA, and the nucleic acid polymerization is RT-qPCR.

In an exemplary embodiment of the present invention, the hot start PCR method using the blocking oligonucleotide of the present invention may be effectively applied to the following cases:

I. A case where there is an attraction phenomenon of the PCR amplification product by non-specific reaction in a monoplex PCR reaction.
II. A case where multiple amplification product occurs by the non-specific reaction in the PCR reaction.
III. A case where efficiency of the PCR reaction is deteriorated by the primer-dimer.
IV. A case where efficiency of the PCR reaction with respect to a specific target having a low amplification rate is attempted to be increased.
V. A case where a confirmation of at least one amplification product (Multiplex PCR) is attempted.
VI. A case where efficiency of the PCR reaction with respect to the specific target in fields such as inspection and disease diagnosis, and the like, is attempted to be increased.
VII. A case where the real time PCR reaction requiring the specificity is performed.
VIII. A case where a primer having a specifically different Tm value is used.
IX. A case where the non-specific amplification product occurs in one-step RT-PCR reaction.

In addition, the composition for polymerizing the nucleic acid as described herein has the following advantages

as compared to various PCR compositions which have been reported in the related art:

1) Since the reaction is performed according to general PCR methods, a long pretreatment time at a high temperature is not required.
2) The composition is economical as compared to the existing compositions for PCR.
3) Since the production of the amplification product by the priming with low stringency is prevented, the composition may be applied to multiplex PCR reaction in which various samples are simultaneously used.

The present invention will be described in more detail with the following exemplary embodiments. The following exemplary embodiments of the present invention have been described to help the understanding of the present invention.
Here, unless technical and scientific terms used herein are defined otherwise, they have meanings understood by those skilled in the art to which the present invention pertains. Known functions and components which obscure the gist of the present invention in the following description and the accompanying drawings will be omitted.

**[Comparative Example 1] Reverse Transcriptase Polymerase Chain Reaction by Short RNA Fragments**

In a reverse-transcriptase polymerase chain reaction synthesizing cDNA from an RNA template, in order to compare a reverse-transcriptase reaction efficiency between DNA oligonucleotide and RNA oligonucleotide, three kinds of single-stranded RNA oligonucleotides each having different length were synthesized.
The synthesized single-stranded RNA was constructed to have random base sequences (6mer; NNN NNN, 9mer; NNN NNN NNN, 12mer; NNN NNN NNN NNN). DNA oligonucleotides having the same base sequences and the same length as the RNA oligonucleotides were synthesized as a control group.
In order to confirm a reverse-transcriptase polymerase chain reaction (RT-PCR) and a polymerase chain reaction (PCR) reaction by the synthesized single-stranded RNA, 10 ng of human total RNA extracted from Hela cell was added to AccuPower RT PreMix (Bioneer Corp., Korea), and base sequences of Human GAPDH gene was targeted.
100 pmole of each of the synthesized single-stranded RNA oligonucleotides was added to the primer used in the reverse-transcriptase polymerase chain reaction (RT-PCR), and the single-stranded DNA oligonucleotides with the same amount were used as a positive control group. The reverse-transcriptase polymerase chain reaction was performed once at 42°C which is an optimal temperature of Accupower RT Premix (Bioneer Corp., Korea) for 60 minutes, and then performed once at 95°C for 5 minutes for inactivation of the reverse-transcriptase enzyme.
5 ul of the product obtained by the reverse-transcriptase polymerase chain reaction (20 ul) was added to Accupower Hotstart PCR Premix (Bioneer Corp., Korea), then a GAPDH forward primer (GAA GGT GAA GGT CGG AGT CAA CG: SEQ ID NO: 1) and a GAPDH reverse primer (AGT CCT TCC ACG ATA CCA AAG TTG: SEQ ID NO: 2) were designed and synthesized with a target of a Human GAPDH gene, and a PCR reaction was performed.
The PCR reaction was performed by repeating one-cycled process including a step at 95°C for 30 seconds, a step at 55°C for 30 seconds, and a step at 72°C for 30 seconds 30 times in total, and pre-degeneration and final elongation were performed at 95°C for 5 minutes, and at 72°C for 5 minutes, respectively. The product obtained by the PCR reaction was subjected to electrophoresis on agarose gel with a DNA molecular marker, then stained with ethidium bromide (EtBr), and a DNA band amplified by the PCR reaction was photographed by a digital camera.
The PCR reaction was performed on a product obtained by using a reverse-transcriptase PreMix (RT PreMix) (Bioneer Corp., Korea) to which a single-stranded DNA was not added, synthesized as a positive control group.
As a result, it could be appreciated that the reverse-transcriptase polymerase chain reaction (RT-PCR) was performed by RNA as well as the synthesized single-stranded DNA. Results thereof were shown in FIG. 1.
In FIG. 1, lanes 1, 2, and 3, are samples to which 100 pmole of oligonucleotides each having a length of 6mer, 9mer and 12mer were sequentially added at the time of performing the reverse-transcriptase polymerase chain reaction (RT-PCR).
The lane N is a blank specimen to which oligonucleotide was not added.
In FIG. 1, A shows a result obtained by addition of RNA oligonucleotide, followed by RT-PCR, and PCR reaction, and B shows a result obtained by addition of DNA oligonucleotide, followed by RT-PCR, and PCR reaction, as a control group.
The lane M is 100 bp DNA Ladder (Bioneer Corp., Korea), and size on the PCR product may be confirmed.
It could be appreciated from FIG. 1 that the RT-PCR was achieved by the RNA oligonucleotide as the sample using the DNA oligonucleotide.

**[Comparative Example 2] Non-Specific RT-PCR and PCR Reaction by Small Fragment RNA**

In order to confirm non-specific RT-PCR and PCR reaction by a small fragment RNA, three kinds of single-stranded RNAs each having random base sequences and different length were synthesized. The synthesized single-stranded RNA was constructed to have random base sequences (6mer; NNN NNN, 9mer; NNN NNN NNN, 12mer; NNN NNN NNN NNN).

In order to confirm the non-specific RT-PCR and PCR reaction by the synthesized single-stranded RNA, 10 ng of human total RNA extracted from Hela cell was added to AccuPower RT PreMix (Bioneer Corp., Korea), and Human beta-actin gene was targeted, and a reverse primer having target base sequences (TAG AAG CAT TTG CGG TGG AC: SEQ ID NO: 3) was used as the primer used in the RT-PCR.

In order to confirm the non-specific RT-PCR by the small fragment RNA, 200 pmole of the synthesized single-stranded RNAs were added, respectively. As a positive control group, a sample to which the synthesized single-stranded RNA was not added was used and was subjected to RT-PCR. The reverse-transcriptase polymerase chain reaction (RT-PCR) was performed once at 42°C which is an optimal temperature of Accupower RT Premix (Bioneer Corp., Korea) for 60 minutes, and then performed at 95°C for 5 minutes once for inactivation of the reverse-transcriptase enzyme.

5 ul of the product obtained by the reverse-transcriptase polymerase chain reaction (20 ul) was added to Accupower Hotstart PCR Premix (Bioneer Corp., Korea), then beta-actin forward primer (AGG ATT CCT ATG TGG GCG AC: SEQ ID NO: 4) and a beta-actin reverse primer (TAG AAG CAT TTG CGG TGG AC: SEQ ID NO: 5) were designed and synthesized with a target of a Human beta-actin gene, and a PCR reaction was performed.

The PCR reaction was performed by repeating one-cycled process including a step at 95°C for 30 seconds, a step at 55°C for 30 seconds, and a step at 72°C for 30 seconds 30 times in total, and pre-degeneration and final elongation were performed at 95°C for 5 minutes, and at 72°C for 5 minutes, respectively. The product obtained by the PCR reaction was subjected to electrophoresis on agarose gel with a DNA molecular marker, then stained with ethidium bromide (EtBr), and a DNA band amplified by the PCR reaction was photographed by a digital camera.

The PCR reaction was performed on a product obtained by using a reverse-transcriptase PreMix (RT PreMix) (Bioneer Corp., Korea) to which a single-stranded DNA was not added, synthesized as a positive control group.

As a result, it could be confirmed that the non-specific reaction occurred on all samples to which the synthesized single-stranded RNA was added. Results thereof were shown in FIG. 2.

In FIG. 2, lanes 1, 2, and 3, are samples to which 200 pmole of the synthesized single-stranded RNA was added at the time of performing the reverse-transcriptase polymerase chain reaction (RT-PCR), and lane 4 is a sample to which the synthesized single-stranded RNA was not added.

Lane 1 is a sample to which the single-stranded RNA having six random base sequences (6mer) was added, and lane 2 is a sample to which the single-stranded RNA having nine random base sequences (9mer) was added, and lane 3 is a sample to which the single-stranded RNA having twelve random base sequences (12mer) was added.

The lane 4 shows a result obtained by not adding the synthesized single-stranded RNA sample, followed by RT-PCR, and PCR reaction, as a positive control group. The lanes 1, 2, and 3 show a result obtained by adding 200 pmole of the synthesized single-stranded RNA sample each having different length, followed by RT-PCR, and PCR reaction. The lane M is 100 bp DNA Ladder (Bioneer Corp., Korea), and size on the PCR product may be confirmed.

As a result, it could be confirmed that the target was not detected by the non-specific reaction in all samples having the single-stranded RNA added thereto regardless of the length, as compared to the sample to which the synthesized single-stranded RNA was not added.

The results mean that the single-stranded RNA functions as a primer except for the target primer in the RT-PCR and PCR reaction, which confirmed that the non-specific reaction is induced.

### [Example 1] Inhibition Effect of Non-Specific RT-PCR of Single-Stranded RNA Terminated by 3'-deoxyadenosine 5'-triphosphate (3'-dATP)

(1) Termination reaction of RNA fragment by poly(A) polymerase and **3'-**deoxyadenosine 5'-triphosphate (3'-dATP)

In order to inhibit the non-specific priming reaction of single-stranded RNAs, poly(A) polymerase(Takara, Japan) and 3'-deoxyadenosine 5'-triphosphate (3'-dATP) were used to perform the termination reaction.

5 ul of 10X Reaction buffer, 0.1 mM 3'-deoxyadenosine 5'-triphosphate (3'-dATP), 2 units of poly(A) polymerase, and 1 nmole of single-stranded RNA having twelve random base sequences (12mer) synthesized in Comparative Example 1 were added, then sterilized distilled water was added to have final volume of 50 ul, followed by reaction at 37°C for 15 minutes.

After the reaction, purification was performed by AccuPower PCR Purification kit (Bioneer Corp., Korea). In the purified product, whether or not 3'-deoxyadenosine 5'-triphosphate (3'-dATP) was synthesized in the single-stranded RNA with molecular weights by Maldi-tof-mass spectrometer (Shimadzu Corp., Japan) was confirmed. The single-stranded RNA having random base sequences had a molecular weight of 3408.2 to 3888.4 MW, and 3'-deoxyadenosine 5'-triphosphate (3'-dATP) had a molecular weight of 491.18 MW.

After 3'-deoxyadenosine 5'-triphosphate (3'-dATP) was synthesized in the single-stranded RNA (12 mer) by using poly(A) polymerase, the molecular weight was measured as 4000 to 4300 MW, which confirmed that 3'-deoxyadenosine 5'-triphosphate was added by poly(A)polymerase. Results thereof were shown in FIG. 3.

(2) Inhibition Effect of Non-Specific RT-PCR of Single-Stranded RNA Terminated by 3'-deoxyadenosine 5'-triphosphate

An RNA oligonucleotide having random base sequences terminated by 3'-deoxyadenosine 5'-triphosphate (3'-dATP) in the same RNA oligonucleotide as Comparative Example 1 above was used with the same template RNA and primer as Comparative Example 1, and by the method (1) as described above. As a control group, a single-stranded RNA oligonucleotide which was not terminated by 3'-deoxyadenosine 5'-triphosphate (3'-dATP) was used.

In order to confirm an inhibition reaction of RT-PCR by the single-stranded RNA terminated by 3'-deoxyadenosine 5'-triphosphate (3'-dATP), 10 ng of human total RNA extracted from Hela cell was added to AccuPower RT PreMix (Bioneer Corp., Korea), and Human GAPDH gene was targeted, and 100 pmole of each of the single-stranded RNA oligonucleotides having 3'-deoxyadenosine added thereto was added to the primer used in the reverse-transcriptase polymerase chain reaction (RT-PCR), and as a positive control group, an RNA oligonucleotide with the same amount but without 3'-deoxyadenosine added thereto was used. The reverse-transcriptase polymerase chain reaction (RT-PCR) was performed once at 42°C which is an optimal temperature of Accupower RT Premix (Bioneer Corp., Korea) for 60 minutes, and then performed at 95°C for 5 minutes once for inactivation of the reverse-transcriptase enzyme.

The RT-PCR and PCR reaction were performed by the same method as Comparative Example 1. The PCR reaction was performed on a product obtained by using a reverse-transcriptase PreMix (RT PreMix) (BIoneer Corp., Korea) containing RNA oligonucleotide to which 3'-deoxyadenosine was not added as a positive control group.

As a result, it could be confirmed that in the sample using the RNA oligonucleotide terminated by 3'-deoxyadenosine, the non-specific RT-PCR and PCR reaction were inhibited. Results thereof were shown in FIG. 4.

In FIG. 4, lanes 1, 2, and 3, are samples in which 100 pmole of single-stranded RNA oligonucleotides each having a length of 6mer, 9mer and 12mer were added in sequence at the time of performing the reverse-transcriptase polymerase chain reaction (RT-PCR).

In FIG. 4, A shows a result obtained by addition of RNA oligonucleotide to which 3'-deoxyadenosine was not added, followed by RT-PCR, and PCR reaction, and B shows a result obtained by addition of RNA oligonucleotide terminated by 3'-deoxyadenosine, followed by RT-PCR, and PCR reaction.

The lane M is 100 bp DNA Ladder (Bioneer Corp., Korea), and size on the PCR product may be confirmed.

As a result, it could be confirmed that the non-specific amplification reaction in the RT-PCR and PCR reaction was inhibited by performing termination by 3'-deoxyadenosine 5'-triphosphate (3'-dATP).

### [Comparative Example 3] Inhibition Phenomenon of Real-Time RT-PCR by RNA Extracted from Human Blood Serum

In order to perform real-time RT-PCR, a template RNA was firstly constructed. Specifically, Hepatitis C virus part was synthesized by gene synthesis method (Biochem. Biophys, Res. Commun 1998, 248, 200-203) and a portion thereof was cloned to pGEM-T-Easy vector (Cat. A1360, Promega Corp., US).

Specifically, 5 ul of 2x rapid ligation buffer (Promega Corp., US), 1 ul of T-easy vector (Promega Corp., US), 1 ul of T4 DNA ligase (Promega Corp., US), and 3 ul (8 ng) of the synthesized gene were put into the same tube and mixed with each other, and placed at a constant temperature of 37°C for 1 hour.

Then, 5 ul of the constant temperature-placed reaction solution was put into 50 ul of *E.coli* competent cell, then left on ice for 40 minutes, followed by culturing at 42°C for 40 seconds, then left on ice again for 2 minutes. After the reaction solution was inoculated into LB plate containing Ampicillin, IPTG and X-gal, followed by culturing at 37°C for 16 hours.

After white colony was taken and cultured in LB liquid medium for about 16 hours, followed by centrifugation to remove a supernatant, and a plasmid DNA was extracted from pellet using AccuPrep Plasmid Prep kit (Bioneer Corp., Korea). Concentration and purity of the plasmid DNA were measured by UV spectrometer (Shimazu Corp., Japan), and it was confirmed that the purity thereof was 1.8 to 2.0, and then, the plasmid DNA was transcribed by MAXIscript In vitro transcription kit (Ambion Corp., US).

After the transcription, concentration and purity were measured by UV spectrometer, and DNA having the purity between 1.8 to 2.0 was used as a template RNA in the subsequent real-time RT-PCR. A copy number of RNA was calculated by the following Equation:

[Equation 1]

$$6.02 \times 10^{23} \times \text{concentration (concentration measured by UV spectrometer g/ml)}/(3015 + 150) \times 340$$

(3015 bp: size of T-vector, 150 bp: size of Hepatitis C virus template RNA)

After calculating the copy number of the template RNA, the RNA was 10 times-diluted with 1x TE buffer (10 mM TrisHCl, pH 8.0, 0.1 mM EDTA), and stored at -70°C before use.

AccuPrep Viral RNA Extraction kit (Bioneer Corp., Korea) was used for extraction of the RNA from the human blood serum (SIGMA-ALDRICH Corp., US), then 200ul of the human blood serum was used for extraction, and purification was performed by using DEPC-D.W so as to have the final volume of 50 ul.

As a control group, RNA was extracted by the same method as the extraction of RNA from the human blood serum, using 200 ul of DEPC-D.W. The RNA extracted from the blood serum was quantified as 2ug by NanoDrop 2000 (Thermo Scientific Corp., US) and then used. The real-time RT-PCR was performed by AccuPower HCV Quantitative RT-PCR kit (Bioneer Corp., Korea).

Constructed template Hepatitis C virus RNAs were added to AccuPower HCV Quantitative RT-PCR kit so as to have the copy number of $10^3$, $10^2$, and $10^1$. Samples extracted using the human blood serum, and DEPC-D.W were put thereinto, together with the template RNA, so as to have the final volume of 50 ul.

The real-time RT-PCR was performed by Exicycler 96 Real-Time Quantitative Thermal Block (Bioneer Corp., Korea), wherein for the reaction condition, one time reaction cycle including the RT-PCR at 50°C for 15 minutes, the pre-degeneration at 95°C for 5 minutes, the degeneration at 95°C for 5 seconds, the annealing at 60°C for 5 seconds, the elongation, and the scanning detecting fluorescence, being performed at the same time, was repeated 45 times in total. Results thereof were shown in FIG. 5.

As a result, it could be confirmed that C(t) in the sample having the human blood serum-extracted RNA added thereto was moved to the right side as compared to the sample having the product extracted by using DEPC-D.W, and fluorescence values were remarkably decreased. From the phenomenon that C(t) was moved to the right side, and the fluorescence values were decreased, it could be appreciated that the RNA extracted from human blood serum caused the non-specific reaction, which is competitively reacted with the HCV-specific RT-PCR, thereby inhibiting amplification. Therefore, it could be appreciated that when the RNA extracted from the human blood serum was added, the real time RT-PCR of HCV was inhibited as compared to the case of using DEPC-D.W.

In FIG. 5, A shows a result obtained by adding the sample extracted from DEPC-D.W, and B shows a result obtained by adding the sample extracted from human blood serum.

**[Example 2] Inhibition Effect of Non-Specific Reaction in Real-Time PCR through RNA Termination Present in Human Blood Serum**

(1) <u>Termination Reaction of RNA by poly(A) polymerase and 3'-deoxyadenosine 5'-triphosphate (3'-dATP)</u>

After RNA was extracted from human blood serum as described in Example 1 above, and 3' terminal of the extracted RNA (3ug) was terminated by poly(A) polymerase and 3'-deoxyadenosine 5'-triphosphate (3'-dATP) (Treatment Group).

It is essential to eliminate 3'-deoxyadenosine 5'-triphosphate (3'-dATP) which is unreacted and remained during RNA termination since it may function as an inhibitor for RT-PCR and PCR reaction.

The RNA terminated by 3'-deoxyadenosine 5'-triphosphate (3'-dATP) was purified by AccuPrep PCR Purification kit (Bioneer Corp., Korea). The RNA of which 3' terminal was not terminated (non-treatment group) was also purified by the same method as described above, and the same amount of each RNA for the treatment group and the non-treatment group was quantified as 2μg as described in Example 1 above, and the all conditions were performed as the same except for performing the termination.

The real-time RT-PCR was performed by AccuPower HCV Quantitative RT-PCR kit (Bioneer Corp., Korea). Constructed template Hepatitis C virus RNAs were added to AccuPower HCV Quantitative RT-PCR kit so as to have the copy number of $10^4$, $10^3$, and $10^2$.

Each of the terminated RNA (treatment group) and the generally extracted RNA (non-treatment group) was put thereinto, together with the template RNA, so as to have the final volume of 50 ul.

The real-time RT-PCR was performed by Exicycler 96 Real-Time Quantitative Thermal Block (Bioneer Corp., Korea), wherein for the reaction condition, one time reaction cycle including the RT-PCR at 50°C for 15 minutes, the pre-degeneration at 95°C for 5 minutes, the degeneration at 95°C for 5 seconds, the annealing at 60°C for 5 seconds, the elongation, and the scanning detecting fluorescence, being performed at the same time, was repeated 45 times in total. Results thereof were shown in FIG. 6.

As a result, it could be confirmed that the inhibition phenomenon of reaction in the real-time RT-PCR was remarkably decreased in the sample using the terminated RNA (FIG. 6C) as compared to the sample using the non-terminated RNA (FIG. 6B).

From the results above, it could be confirmed that the RNA could function as a primer in the RT-PCR and PCR reaction, and the RNA was previously prevented from functioning as the primer, such that the non-specific amplification was inhibited, whereby the RT-PCR and PCR reaction were capable of being improved.

In FIG. 6, A shows a result obtained by adding the template RNA only as a template, B shows a result obtained by adding RNA generally extracted from the human blood serum to the template RNA, and C shows a result obtained by adding the RNA extracted from the human blood serum and terminated to the template RNA.

(2) Removal of Hydrolysis of 3'-deoxyadenosine 5'-triphosphate (3'-dATP) Using Alkaline Phosphatase (CIP)

3'-deoxyadenosine 5'-triphosphate (3'-dATP) used in the termination of the RNA in the above (1) remained after the termination reaction, wherein it is preferable to eliminate the remained 3'-dATP after the RNA termination since it functions as an inhibitor in the RT-PCR and PCR reaction.

In Example 1 and the above (1), AccuPrep PCR Purification kit (Bioneer Corp., Korea) was used to eliminate 3'-deoxyadenosine 5'-triphosphate (3'-dATP).

In order to inhibit the activity of 3'-deoxyadenosine 5'-triphosphate (3'-dATP) without using the purification kit, CIP (Sigma-Aldrich) was used.

After the RNA was terminated by the same method as the above (1), a sample treated by CIP to inhibit 3'-deoxyadenosine 5'-triphosphate (3'-dATP) was used, and a sample in which the RNA was terminated by 3'-deoxyadenosine 5'-triphosphate (3'-dATP) and then 3'-deoxyadenosine 5'-triphosphate (3'-dATP) was contained without purification, and a sample from which 3'-deoxyadenosine 5'-triphosphate (3'-dATP) was removed by AccuPrep PCR Purification kit, were used as a control group.

CIP was inactivated with high temperature and pH variation, and therefore, inactivated in RT-PCR and PCR reaction, thereby minimizing the reaction. Then, the real-time RT-PCR was performed by the same condition as the above (1). Results thereof were shown in FIG. 7.

In FIG. 7, A shows a result obtained by using a sample in which the fragment RNA was terminated, and then 3'-deoxyadenosine 5'-triphosphate (3'-dATP) was hydrolyzed by CIP, B and C as a control group show results obtained by using a sample in which the RNA was terminated by 3'-deoxyadenosine 5'-triphosphate (3'-dATP) and then 3'-deoxyadenosine 5'-triphosphate (3'-dATP) was contained without purification, and C as a sample from which 3'-deoxyadenosine 5'-triphosphate (3'-dATP) was removed by AccuPrep PCR Purification kit, respectively.

As a result, it could be confirmed that the RT-PCR and PCR reaction were inhibited by 3'-deoxyadenosine 5'-triphosphate (3'-dATP).

Meanwhile, it could be confirmed that the RT-PCR and PCR reaction were not inhibited in the specimen from which the remained 3'-deoxyadenosine 5'-triphosphate (3'-dATP) was removed by AccuPrep PCR Purification kit and CIP.

It could be confirmed from the above results that the non-specific self-priming by the RNA itself contained in a large number of RNAs could be removed by treating the purified nucleic acid with 3'-deoxyadenosine 5'-triphosphate (3'-dATP) of the Example, and nucleic acids capable of detecting the target nucleic acid could be obtained with high sensitivity.

In addition, it could be confirmed from the above results that the self-priming of the template nucleic acid inhibiting the amplification reaction of the target nucleic acid in the PCR reaction or the real-time quantitative PCR reaction for detecting a trace nucleic acid was basically eliminated, such that the target nucleic acid only could be precisely amplified and detected, or a concentration of the target nucleic acid only could be precisely measured.

**[Example 3] Inhibition Effect of Non-Specific RT-PCR by RNA Self-Priming through RNA Termination**

(1) Termination reaction of RNA fragment by poly(A) polymerase and 3'-deoxyadenosine 5'-triphosphate (3'-dATP)

The RNA was extracted from Hela C cell which is a kind of human cell by RNA extraction kit as the same as Example 1 above. 3'-terminal of the extracted RNA was terminated by using poly(A) polymerase and 3'-deoxyadenosine 5'-triphosphate (3'-dATP) (C, D).

In order to eliminate the remained 3'-deoxyadenosine 5'-triphosphate (3'-dATP), the terminated RNA was purified by AccuPrep PCR Purification kit (Bioneer Corp., Korea). The RNA of which 3' terminal was not terminated (A and B) were purified by the same method as described above for comparison under the same condition. In order to compare the effect of the hot start RT-PCR, RT-PCR having hot start function was performed on B and D, and RT-PCR without hot start function was performed on A and C.

5μg of each RNA was used, and AccuPower RocketScript RT Premix (Bioneer Corp., Korea) was used for the general PCR, and for hot start RT-PCR, pyrophosphate and phosphatase (Korean Patent No. 10-1098764) were added to AccuPower RocketScript RT Premix (Bioneer Corp., Korea), then distilled water was added so as to have the final volume of 20 ul, and the hot start RT-PCR was performed. In order to confirm the effect of the RNA self-priming, any kinds of primers required for the RT-PCR were not added thereto.

The RT-PCR was performed by Mygenie 96 Gradient Thermal Block (Bioneer Corp., Korea), and in order to confirm the effect of the RNA self-priming on the reaction mixing process, incubation was performed at 25°C for 20 minutes.

Then, in order to confirm whether or not the RT-PCR by the RNA self-priming progressed, the real-time PCR was performed on the target human GAPDH gene by using each of the RT-PCR products as the template, and using AccuPower Dualstar qPCR Premix (Bioneer Corp., Korea) (Forward Primer 5'-CGTGGAAGGACTCATGACCACA-3': SEQ ID NO: 6, Reverse Primer GCCTTGGCAGCGCCAGTAGA-3': SEQ ID NO: 7, and GAPDH probe 5'-CTGTGGATGGCCCCTCCGGGAAA-3': SEQ ID NO: 8 , each has concentration of 10 nM).

The real-time PCR was performed by Exicycler 96 Real-Time Quantitative Thermal Block (Bioneer Corp., Korea), wherein for the reaction condition, one time reaction cycle including the pre-degeneration at 95°C for 5 minutes, the degeneration at 95°C for 5 seconds, the annealing at 60°C for 5 seconds, elongation, and scanning detecting fluorescence, being performed at the same time, was repeated 45 times in total.

Results thereof were shown in FIG. 8. It could be confirmed that even though any primer was not added, cDNA was produced in the non-terminated RNAs (A and B) by the detection of the human GAPDH gene. As the above result, since cDNA was synthesized by the self-priming, the amplification was achieved in the PCR.

In addition, even though the hot start RT-PCR prevents the non-specific cDNA synthesis and the subsequent PCR amplification, it is not completely inhibited, but the RT-PCR by the RNA self-priming is partially inhibited, such that the Ct value was moved to the right side by about 5, thereby inhibiting the reaction with a ratio of 1/30.

However, it could be appreciated from the samples having the RNA of which 3' terminal was terminated (C and D) that the amplification in all RT-PCR was not achieved up to by 45 cycles, such that cDNA was not synthesized at all.

It could be confirmed from the above result that many non-specific RT-PCRs occurred at the time of mixing RT-PCR reactants, and thus, a number of non-specific cDNAs were produced. In addition, it could be confirmed from the above results that unwanted reactions could not be inhibited only by the hot start RT-PCR, and eventually, the non-specific RT-PCR by the RNA self-priming could be completely inhibited by prevention using the termination reaction of the present invention.

In FIG. 8, the non-terminated RNA was used in A and B, and the terminated RNA was used in C and D. The general RT reaction was performed in A and C, and the hot start RT reaction was performed in B and D.

**[Example 4] Inhibition Effect of Non-Specific Reaction by Various Kinds of Modified Blocking Oligonucleotide**

After various kinds of substituent groups were applied to 3' terminal of the blocking oligonucleotide, an effect of the hot start reaction on the blocking oligonucleotide was confirmed. The used substituent groups were shown in the following Table 2. The PCR reaction was performed by repeating one-cycled process including a step at 95°C for 20 seconds, a step at 55°C for 40 seconds, and a step at 72°C for 60 seconds 30 times in total, and the pre-degeneration and final elongation were performed at 94°C for 5 minutes, and at 72°C for 5 minutes, respectively. Results thereof were shown in FIG. 12.

In FIG. 12, lanes 1, 2, and 3 show results of using primer couples having base sequences of P55/P63(447 bp), P55/P73(1,082 bp) and P55/P83(1,29 6bp) shown in Table 1, respectively. In addition, A shows a result obtained by using a hot start PCR premix having a hot start function as a positive control group, and B, C, D, E, F and G as negative control groups show results obtained by using each instant blocking oligonucleotide shown in the following Table 2 in sequence in a PCR premix not having the hot start function, followed by the PCR reaction. The lane M shows a 100 bp DNA ladder capable of differentiating a DNA size.

[Table 2]

| No. | Type of modification | chemical Formula |
|---|---|---|
| 1 | 3' amine | 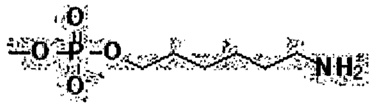 |
| 2 | 3' phosphate | 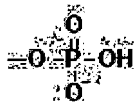 |
| 3 | 3' C3-space | 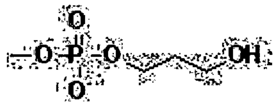 |
| 4 | 3' C5-space | 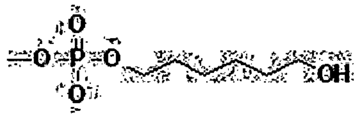 |
| 5 | 3' C12-space | 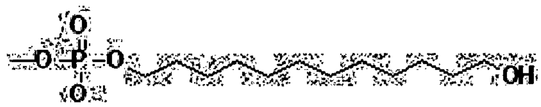 |
| 6 | 3' C18-space | 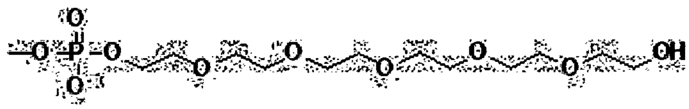 |

As a result, it could be confirmed that even though there are any kinds of chemicals capable of blocking the DNA polymerase at 3' terminal of the blocking oligonucleotide having complementary base sequences to the primer, the non-specific amplification reaction could be inhibited (FIG. 12).

**[Example 5] Inhibition Effect of Primer-Dimer by Blocking Oligonucleotide in Real Time PCR Reaction**

In order to confirm the hot start effect by the PCR composition containing the blocking oligonucleotide in the real-time PCR detection, a composition was prepared by adding a fluorescent material (Greenstar™, Bioneer Corp.) to 2x PCR Premix solution (containing 10 mM TrisHCl pH 9.0, 50 mM KC1, 2.0 mM $MgCl_2$, 250 μM of each of four kinds of dNTPs, 1U Taq. DNA polymerase, 0.01% Tween 20 and stabilizer) so as to be 0.3X for each reaction of 20 μℓ, and then adding 20 pmole of the blocking oligonucleotide which is the same concentration of the forward primer. As a control group, the fluorescent material was added to the 2x PCR Premix solution so as to be 0.25X for each reaction of 20 μℓ. The fluorescent material is a fluorescent material which is capable of being conveniently analyzed by measuring fluorescent amount generated by intercalation between double-stranded DNA occurred at the time of amplifying the template DNA without fluorescent probe having specific base sequences. 5 μℓ of cDNA obtained by adding 100 ng of total RNA extracted from human cell to AccuPower CycleScript RT PreMix (Bioneer Corp.), followed by reaction at 42°C for 1 hour, and then inactivating the reverse transcriptase enzyme at 95°C for 5 minutes, was used as the template DNA. The primer and the blocking oligonucleotide described in the following Table 3 were used with each concentration of 20 pmole for each reaction of 20 μℓ.

[Table 3]

| Name | Primer | Lengt h | Tm(°C) | Sequence | Seq ID No. |
|---|---|---|---|---|---|
| GM501 qPCR | Forward Primer | 20mer | 55.2 | GAC GTC CGC ATC TTG AAT TG | 9 |
| | Reverse Primer | 20mer | 55.2 | TTC CCA CGA TTA GGA GCA CA | 10 |
| | F-Blocking Oligo Primer | 17mer | 45.8 | CAA TTC AAG ATG CGG AC | 11 |

The real-time PCR reaction was performed by Exicycler 96 Real-Time Quantitative Thermal Block (Bioneer Corp., Korea), wherein for the reaction condition, one time reaction cycle including the pre-degeneration at 94°C for 5 minutes, the degeneration at 95°C for 10 seconds, the annealing at 60°C for 15 seconds, and the elongation, being performed at the same time, was repeated 45 times in total. Then, a dissociation step was performed, then a melting curve on the amplification product was prepared to confirm PCR reactivity and specificity on the PCR amplification product. Results thereof were shown in FIG. 13.

As a result, threshold cycle values (Ct) with respect to the line I and the line II were 23.89 C(t) of the line I and 29.5C(t) of the line II (FIG. 13). In the line I, the C(t) value was moved to the left side as compared to that of the line II, and the reason is because the line II containing the blocking oligonucleotide showed the only one fluorescent peak indicating that precise amplification product was only produced; meanwhile, the line I not containing the blocking oligonucleotide showed two fluorescent peaks indicating that two amplification products were produced, as shown in the melting curve. This means that the primer-dimer amplification product or the non-specific amplification product having a size smaller than that of the desired PCR amplification product was produced. In addition, the melting temperature (Tm) on the precise PCR amplification product was confirmed as 85.5°C in the experimental group (line II) and the control group (line I), and the melting temperature (Tm) on the primer-dimer or the non-specific amplification product was confirmed to be 75.5°C which was lower than that of the precise PCR amplification product.

It could be appreciated from the above-results that in the control group, the primer-dimer in addition to the amplification product was produced to have low specificity, and in contrast, the instant hot start PCR method used in the present invention could effectively recover the PCR reaction inhibiting the non-specific reaction shown at the low temperature in the real-time PCR reaction and the PCR reaction, thereby having advantages of the hot start method in that the non-specific PCR amplification product is inhibited from being produced, and precise results are confirmed.

[Industrial Applicability]

With the method for treating a nucleic acid according to the present invention, the nucleic acid polymerization terminator is added to the template nucleic acid used in detecting the nucleic acid using the nucleic acid polymerization such as the PCR reaction, the real time quantitative PCR reaction, or the like, for detecting a trace of nucleic acid, such that the non-specific self-priming inhibiting the amplification reaction of the target nucleic acid and the subsequent non-specific amplification may be basically eliminated, whereby a false-positive amplification may be eliminated, and only the target nucleic acid, particularly, the trace of nucleic acid may be precisely detected.

## Claims

1. A method for treating a nucleic acid before amplifying the nucleic acid using a nucleic acid polymerization reaction to prevent a non-specific priming of the nucleic acid, the method comprising:

   (1) terminating 3'-terminal of the nucleic acid by reacting a reaction mixture comprising nucleic acid, nucleic acid polymerase, an enzyme reaction buffer, and a nucleic acid polymerization terminator inhibiting an elongation of 3' terminal of the nucleic acid; and
   (2) inactivating or eliminating non-reacted free nucleic acid polymerization terminator from the reaction mixture.

2. The method according to claim 1, wherein the nucleic acid polymerization reaction is initiated by priming.

3. The method according to claim 2, wherein the nucleic acid polymerization reaction is at least one selected from the group consisting of RNA-dependent RNA-polymerase reaction, DNA dependent RNA-polymerase reaction, RNA-dependent DNA-polymerase reaction, and DNA dependent DNA polymerase reaction.

4. The method according to claim 1, wherein the nucleic acid in step (1) is separated and purified so as to be capable of termination reaction.

5. The method according to claim 1, wherein the nucleic acid is RNA or DNA, and the nucleic acid polymerase is at least one selected from the group consisting of RNA-dependent RNA-polymerase, RNA-dependent DNA-polymerase, DNA-polymerase, and RNA-polymerase.

6. The method according to claim 5, wherein the RNA-dependent RNA-polymerase is NS5 enzyme derived from Flavivirus.

7. The method according to claim 5, wherein the RNA-dependent DNA -polymerase is at least one selected from the group consisting of MMLV reverse transcriptase, AMV reverse transcriptase and HIV reverse transcriptase.

8. The method according to claim 5, wherein the RNA-polymerase is Poly(A) polymerase as 3'terminal transferase.

9. The method according to claim 1, wherein the nucleic acid polymerization terminator is a nucleic acid-like compound which is activated as a triphosphate form capable of functioning on the nucleic acid polymerase, without 3' hydroxyl group.

10. The method according to claim 9, wherein the nucleic acid-like compound is at least one selected from the group consisting of 2'3'-dideoxynucleoside 5'-triphosphate, 3'-deoxyadenosine 5'-triphosphate, 3'-azido-3'-deoxythymidine 5'-triphosphate, 1-β-d-arabinofuranosylnucleoside 5'-triphosphate, acyclo-guanosine triphosphate, 3'-amino-3'-deoxynucleoside 5'-triphosphate, and 3'-fluoro-3'-deoxynucleoside 5'-triphosphate.

11. The method according to claim 10, wherein the deoxynucleoside triphosphate is at least one selected from the group consisting of guanosine, adenosine, thymidine, uridine, and cytidine.

**12.** The method according to claim 9, wherein inactivating nucleic acid polymerization terminator in the step 2) is phospho-bond hydrolase, which hydrolyzing triphosphate.

**13.** The method according to claim 12, wherein the phospho-bond hydrolase is at least one selected from the group consisting of alkaline phosphatases derived from Escherichia coli, calf small intestine and shrimp, and Autotaxin.

**14.** The method according to claim 1, wherein the step (2) eliminating nucleic acid polymerization terminator is achieved by using gel filtration, or caotropic agent.

**Patentansprüche**

**1.** Verfahren zur Behandlung einer Nucleinsäure vor Amplifizieren der Nucleinsäure unter Verwendung einer Nucleinsäurepolymerisationsreaktion, um ein nicht-spezifisches Priming der Nucleinsäure zu verhindern, wobei das Verfahren umfasst:

(1) Terminieren des 3'-Terminus der Nucleinsäure durch Umsetzen eines Reaktionsgemisches, umfassend Nucleinsäure, Nucleinsäurepolymerase, einen Enzymreaktionspuffer und einen Nucleinsäurepolymerisationsterminator, der eine Verlängerung des 3'-Terminus der Nucleinsäure inhibiert, und
(2) Inaktivieren oder Eliminieren von nicht umgesetztem freien Nucleinsäurepolymerisationsterminator aus dem Reaktionsgemisch.

**2.** Verfahren gemäß Anspruch 1, wobei die Nucleinsäurepolymerisationsreaktion durch Priming initiiert wird.

**3.** Verfahren gemäß Anspruch 2, wobei die Nucleinsäurepolymerisationsreaktion wenigstens eine, ausgewählt aus der Gruppe, bestehend aus RNA-abhängiger RNA-Polymerasereaktion, DNA-abhängiger RNA-Polymerasereaktion, RNA-abhängige DNA-Polymerasereaktion und DNA-abhängiger DNA-Polymerasereaktion, ist.

**4.** Verfahren gemäß Anspruch 1, wobei die Nucleinsäure in Schritt (1) abgetrennt und gereinigt wird, sodass sie zur Terminationsreaktion geeignet ist.

**5.** Verfahren gemäß Anspruch 1, wobei die Nucleinsäure RNA oder DNA ist und die Nucleinsäurepolymerase wenigstens eine, ausgewählt aus der Gruppe, bestehend aus RNA-abhängiger RNA-Polymerase, RNA-abhängiges DNA-Polymerase, DNA-Polymerase und RNA-Polymerase, ist.

**6.** Verfahren gemäß Anspruch 5, wobei die RNA-abhängige RNA-Polymerase aus Flavivirus stammendes NS5-Enzym ist.

**7.** Verfahren gemäß Anspruch 5, wobei die RNA-abhängige DNA-Polymerase wenigstens eine, ausgewählt aus der Gruppe, bestehend aus MMLV-reverser Transkriptase, AMV-reverser Transkriptase und HIV-reverser Transkriptase, ist.

**8.** Verfahren gemäß Anspruch 5, wobei die RNA-Polymerase Poly(A)-polymerase als 3'-terminale Transferase ist.

**9.** Verfahren gemäß Anspruch 1, wobei der Nucleinsäurepolymerisationsterminator eine Nucleinsäure-artige Verbindung ist, welche als Triphosphatform aktiviert wird, die in der Lage ist, als Nucleinsäurepolymerase zu fungieren, und zwar ohne 3'-Hydroxylgruppe.

**10.** Verfahren gemäß Anspruch 9, wobei die Nucleinsäure-artige Verbindung wenigstens eine, ausgewählt aus der Gruppe, bestehend aus 2'3'-Didesoxynucleosid-5'-triphosphat, 3'-Desoxyadenosin-5'-triphosphat, 3'-Azido-3'-desoxythymidin-5'-triphosphat, 1-β-d-Arabinofuranosylnucleosid-5'-triphosphat, Acyclo-guanosintriphosphat, 3'-Amino-3'-desoxynucleosid-5'-triphosphat und 3'-Fluor-3'-desoxynucleosid-5'-triphosphat, ist.

**11.** Verfahren gemäß Anspruch 10, wobei das Desoxynucleosidtriphosphat wenigstens eins, ausgewählt aus der Gruppe, bestehend aus Guanosin, Adenosin, Thymidin, Uridin und Cytidin, ist.

**12.** Verfahren gemäß Anspruch 9, wobei inaktivierender Nucleinsäurepolymerisationsterminator in Schritt 2) Phosphogebundene Hydrolase ist, welche Triphosphat hydrolysiert.

13. Verfahren gemäß Anspruch 12, wobei die Phosphobindungshydrolase wenigstens eine, ausgewählt aus der Gruppe, bestehend aus alkalkischen Phosphatasen, die aus Escherichia coli, Kälberdünndarm und Shrimps stammen, und Autoxin, ist.

14. Verfahren gemäß Anspruch 1, wobei der Schritt (2) Eliminieren von Nucleinsäurepolymerisationsterminator durch Verwendung von Gelfiltration oder chaotropem Agens erreicht wird.

**Revendications**

1. Procédé de traitement d'un acide nucléique avant amplification de l'acide nucléique à l'aide d'une réaction de polymérisation d'acide nucléique pour empêcher un amorçage non spécifique de l'acide nucléique, le procédé comprenant :

   (1) la terminaison de l'extrémité 3' de l'acide nucléique en faisant réagir un mélange de réaction comprenant de l'acide nucléique, une polymérase d'acide nucléique, une solution tampon de réaction d'enzyme et un terminateur de polymérisation d'acide nucléique inhibant un allongement de l'extrémité 3' de l'acide nucléique ; et
   (2) l'inactivation ou l'élimination d'un terminateur de polymérisation d'acide nucléique libre n'ayant pas réagi du mélange de réaction.

2. Procédé selon la revendication 1, dans lequel la réaction de polymérisation d'acide nucléique est débutée par amorçage.

3. Procédé selon la revendication 2, dans lequel la réaction de polymérisation d'acide nucléique est au moins l'une choisie dans le groupe consistant en une réaction d'ARN-polymérase ARN-dépendante, une réaction d'ARN-polymérase ADN-dépendante, une réaction ADN-polymérase ARN-dépendante et une réaction ADN-polymérase ADN-dépendante.

4. Procédé selon la revendication 1, dans lequel l'acide nucléique à l'étape (1) est séparé et purifié de façon à être capable d'une réaction de terminaison.

5. Procédé selon la revendication 1, dans lequel l'acide nucléique est un ARN ou un ADN, et la polymérase d'acide nucléique est au moins l'une choisie dans le groupe consistant en l'ARN-polymérase ARN-dépendante, l'ARN-polymérase ADN-dépendante, l'ADN-polymérase et l'ARN polymérase.

6. Procédé selon la revendication 5, dans lequel l'ARN-polymérase ARN-dépendante est l'enzyme NS5 dérivée d'un Flavivirus.

7. Procédé selon la revendication 5, dans lequel l'ARN-polymérase ADN-dépendante est au moins l'une choisie dans le groupe consistant en une transcriptase inverse Mo MLV, une transcriptase inverse de VMA et une transcriptase inverse de VIH.

8. Procédé selon la revendication 5, dans lequel l'ARN-polymérase est la poly(A) polymérase en tant que transférase terminale 3'.

9. Procédé selon la revendication 1, dans lequel le terminateur de polymérisation d'acide nucléique est un composé de type acide nucléique qui est activé sous forme de triphosphate capable de fonctionner sur la polymérase d'acide nucléique, sans groupe hydroxyle 3'.

10. Procédé selon la revendication 9, dans lequel le composé de type acide nucléique est au moins l'un choisi dans le groupe consistant en le 2'3'-didésoxynucléoside 5'-triphosphate, le 3'-désoxyadénosine 5'-triphosphate, le 3'-azido-3'-désoxythymidine 5-triphosphate, le 1-β-d-arabinofuranosylnucléoside 5'triphosphate, l'acyclo-guanosine triphosphate, le 3'-amino-3'-désoxynucléoside 5'-triphosphate et le 3'-fluoro-3-désoxynucléoside 5'-triphosphate.

11. Procédé selon la revendication 10, dans lequel le désoxynucléoside triphosphate est au moins l'une choisie dans le groupe consistant en la guanosine, l'adénosine, la thymidine, l'uridine et la cytidine.

12. Procédé selon la revendication 9, dans lequel l'inactivation d'un terminateur de polymérisation d'acide nucléique à

l'étape 2) est une hydrolase de liaison phospho, qui hydrolyse le triphosphate.

**13.** Procédé selon la revendication 12, dans lequel l'hydrolase de liaison phospho est au moins l'une choisie dans le groupe consistant en des phosphatases alcalines dérivées *d'Escherichia coli,* de l'intestin grêle du veau et de la crevette, et de l'autotaxine.

**14.** Procédé selon la revendication 1, dans lequel l'étape (2) d'élimination de terminateur de polymérisation d'acide nucléique est accomplie par l'utilisation d'une filtration sur gel ou un agent chaotrope.

Fig. 1

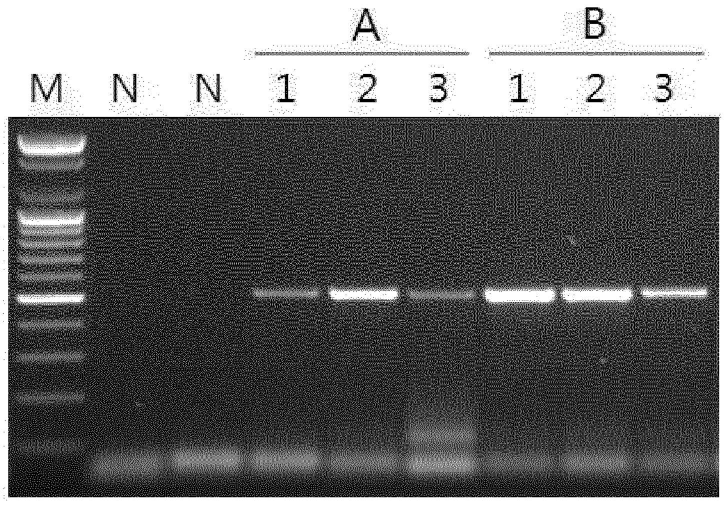


1: 6mer
2: 9mer
3: 12mer

FIG. 2

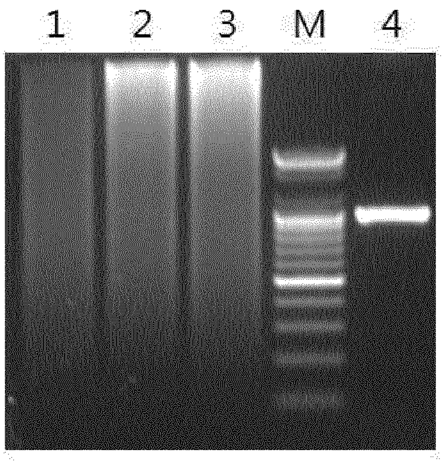


1: 6mer
2: 9mer
3: 12mer
4: Positive Control

FIG. 3

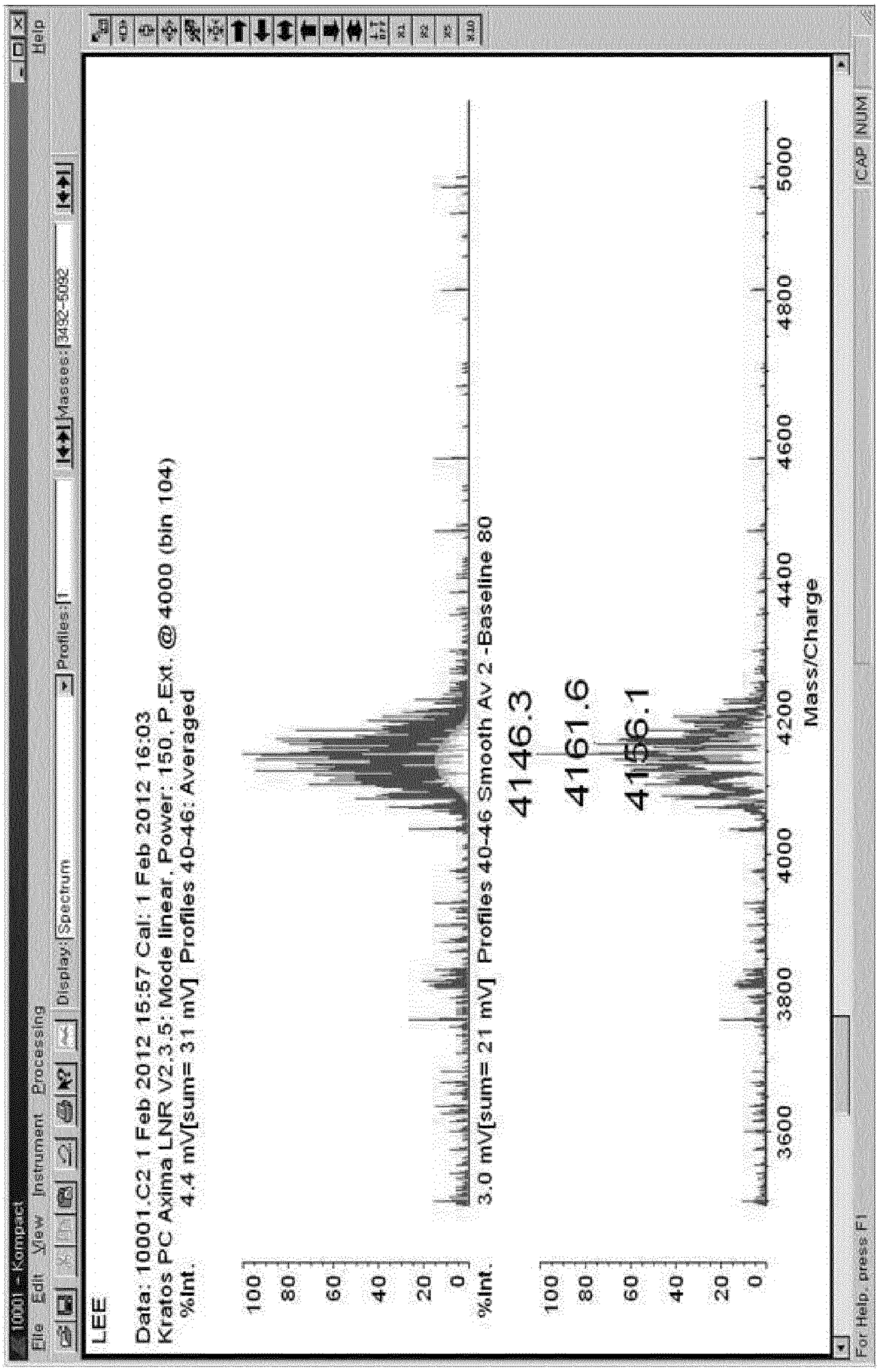

LEE
Data: 10001.C2 1 Feb 2012 15:57 Cal: 1 Feb 2012 16:03
Kratos PC Axima LNR V2.3.5: Mode linear, Power: 150, P.Ext. @ 4000 (bin 104)
%Int. 4.4 mV[sum= 31 mV] Profiles 40-46: Averaged
%Int. 3.0 mV[sum= 21 mV] Profiles 40-46 Smooth Av 2 -Baseline 80
4146.3
4161.6
4156.1
Mass/Charge

FIG. 4

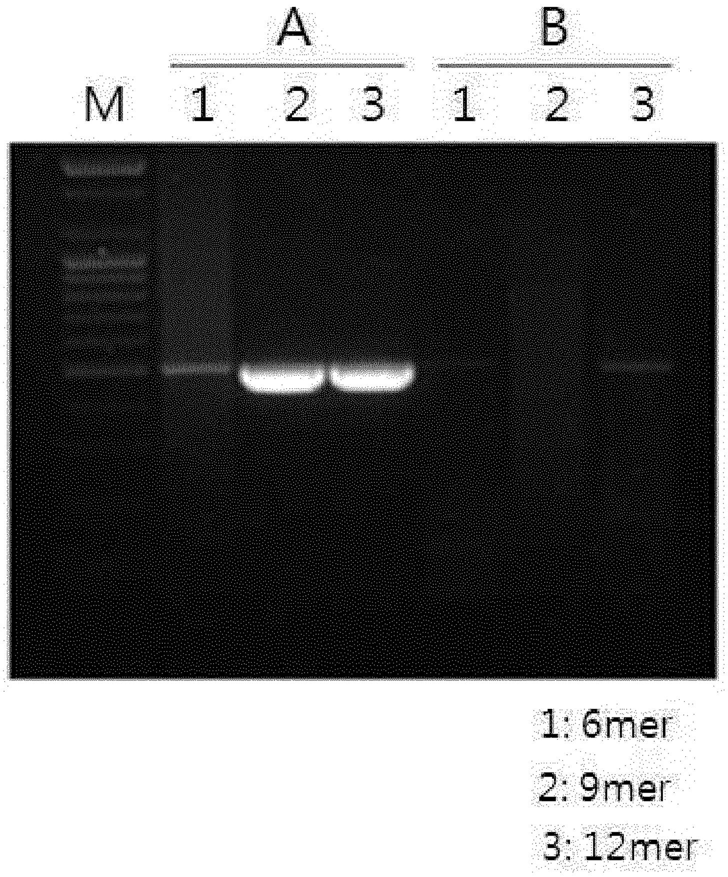

FIG. 5

A

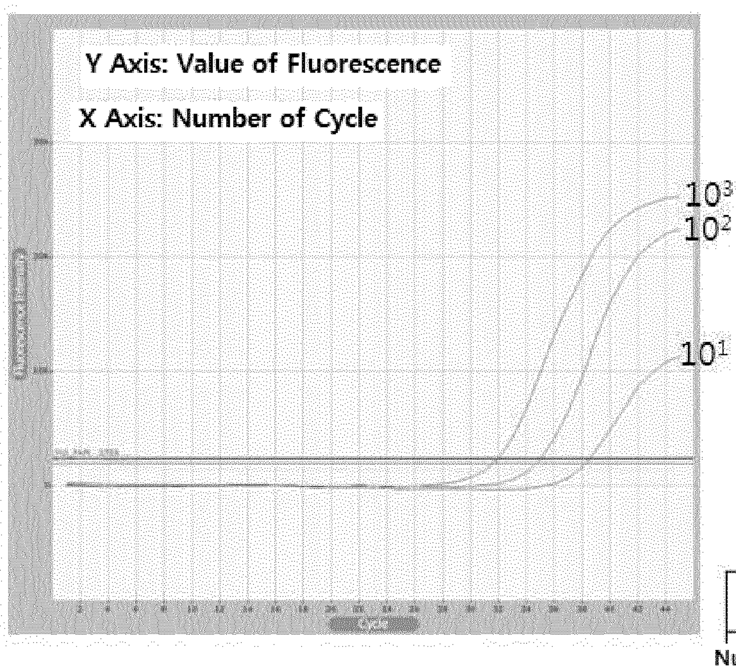


B

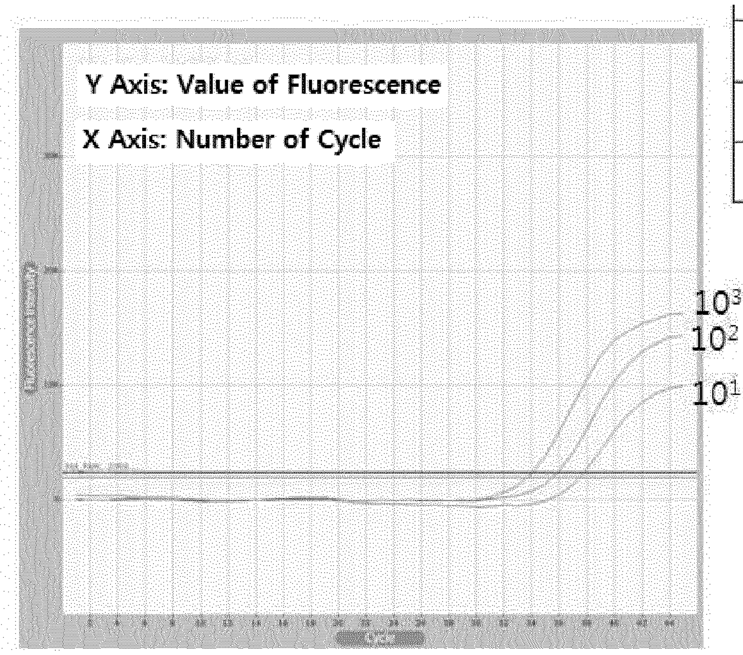


| | C(t) | |
|---|---|---|
| Number of Copy | DEPC_D.W | Serum |
| 10 | 38.52 | 38.77 |
| 100 | 35.03 | 35.88 |
| 1000 | 31.97 | 34.03 |

FIG. 6

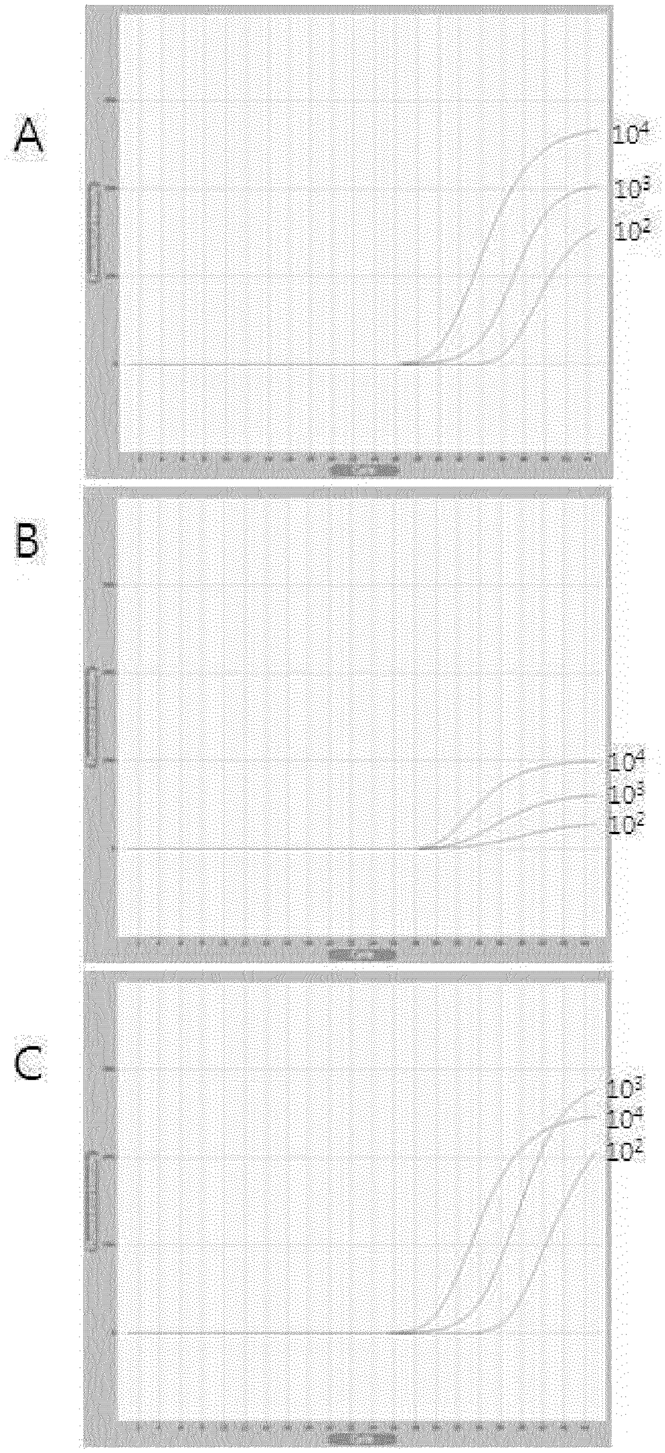


| Number of Copy | C(t) | | |
|---|---|---|---|
| | A | B | C |
| 100 | 35.94 | 36.33 | 36.04 |
| 1000 | 32.04 | 32.78 | 31.61 |
| 10000 | 29.11 | 30.51 | 28.93 |

FIG. 7

A

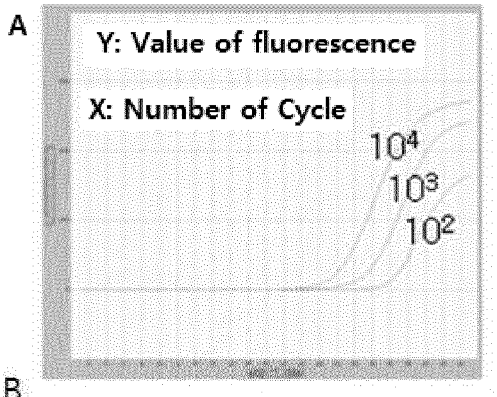


B

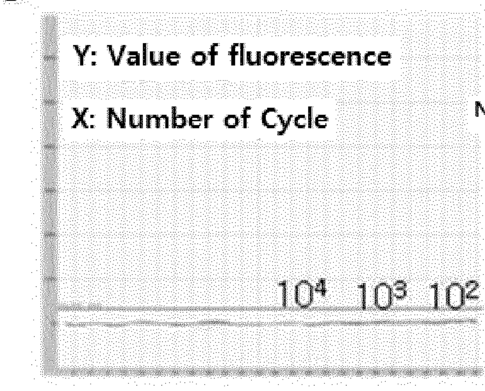


| | C(t) | | |
|---|---|---|---|
| Numebr of Copy | A | B | C |
| 100 | 35.2 | — | 34.12 |
| 1000 | 33.08 | — | 32.04 |
| 10000 | 29.11 | — | 28.39 |

C

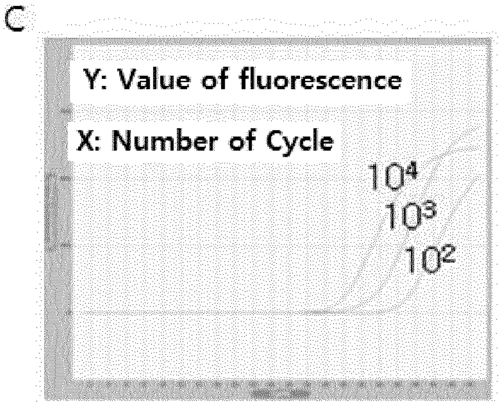

FIG. 8

| | C(t) |
|---|---|
| A | 32.3/32.2 |
| B | 36.5/37.8 |
| C | — |
| D | — |

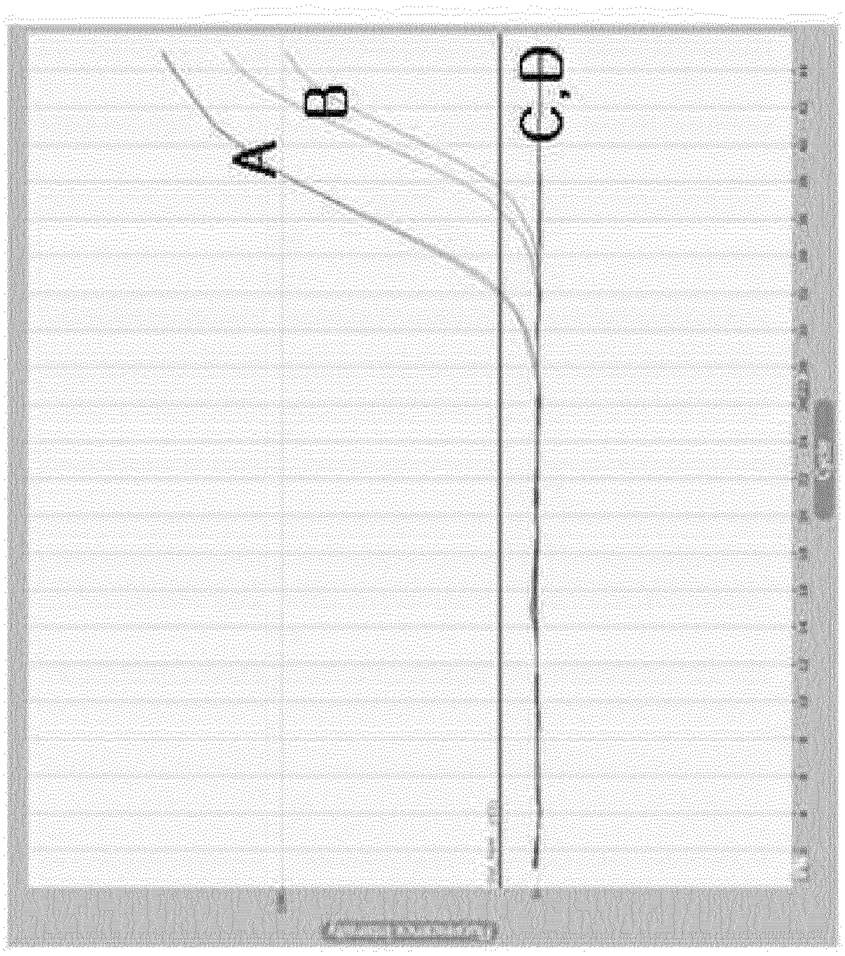

FIG. 9

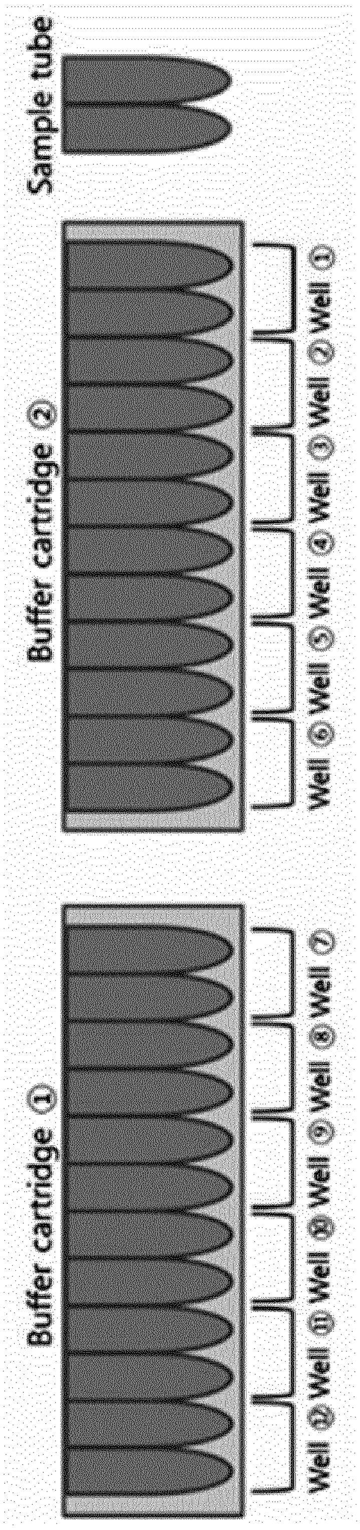
Sample tube
Buffer cartridge ②
Well ① Well ② Well ③ Well ④ Well ⑤ Well ⑥
Buffer cartridge ①
Well ⑦ Well ⑧ Well ⑨ Well ⑩ Well ⑪ Well ⑫

FIG. 10

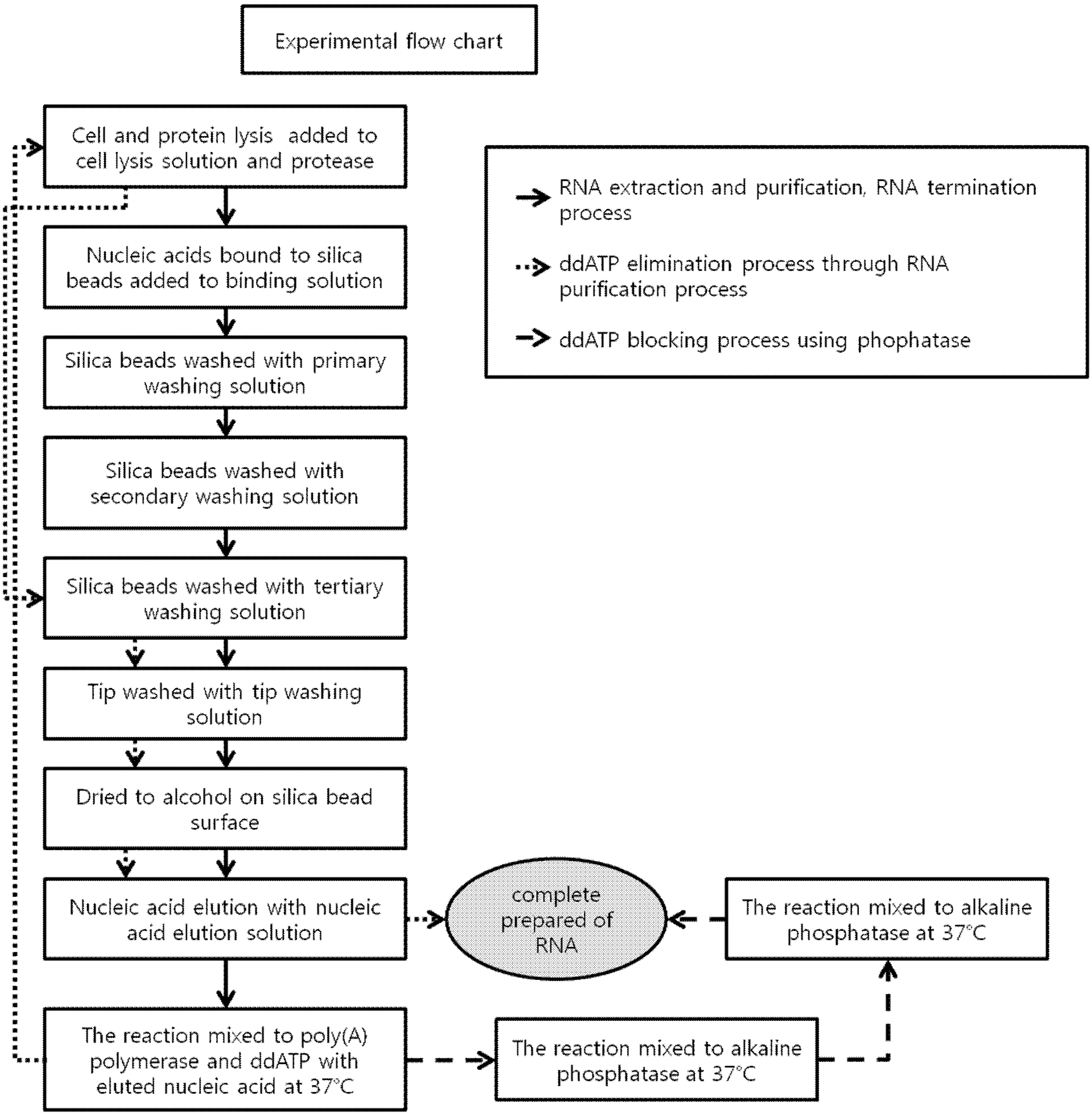
Experimental flow chart
Cell and protein lysis added to cell lysis solution and protease
Nucleic acids bound to silica beads added to binding solution
Silica beads washed with primary washing solution
Silica beads washed with secondary washing solution
Silica beads washed with tertiary washing solution
Tip washed with tip washing solution
Dried to alcohol on silica bead surface
Nucleic acid elution with nucleic acid elution solution
The reaction mixed to poly(A) polymerase and ddATP with eluted nucleic acid at 37°C
The reaction mixed to alkaline phosphatase at 37°C
complete prepared of RNA
The reaction mixed to alkaline phosphatase at 37°C
RNA extraction and purification, RNA termination process
ddATP elimination process through RNA purification process
ddATP blocking process using phophatase

FIG. 11

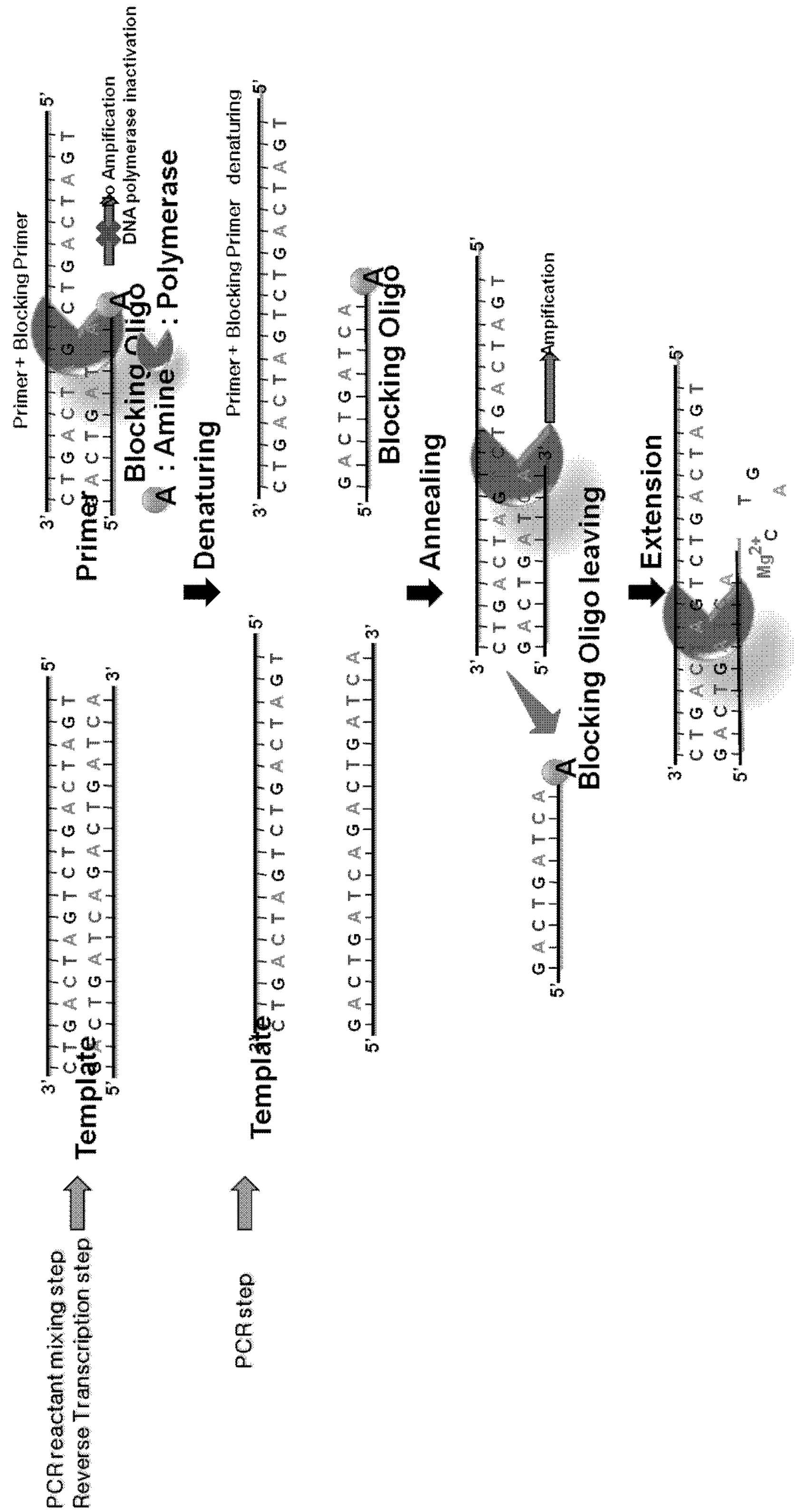

PCR reactant mixing step
Reverse Transcription step
PCR step
Template
Primer
Primer + Blocking Primer
Blocking Oligo
A : Amine
: Polymerase
No Ampification
DNA polymerase inactivation
Denaturing
Primer + Blocking Primer denaturing
Annealing
Ampification
Blocking Oligo leaving
Extension

FIG. 12

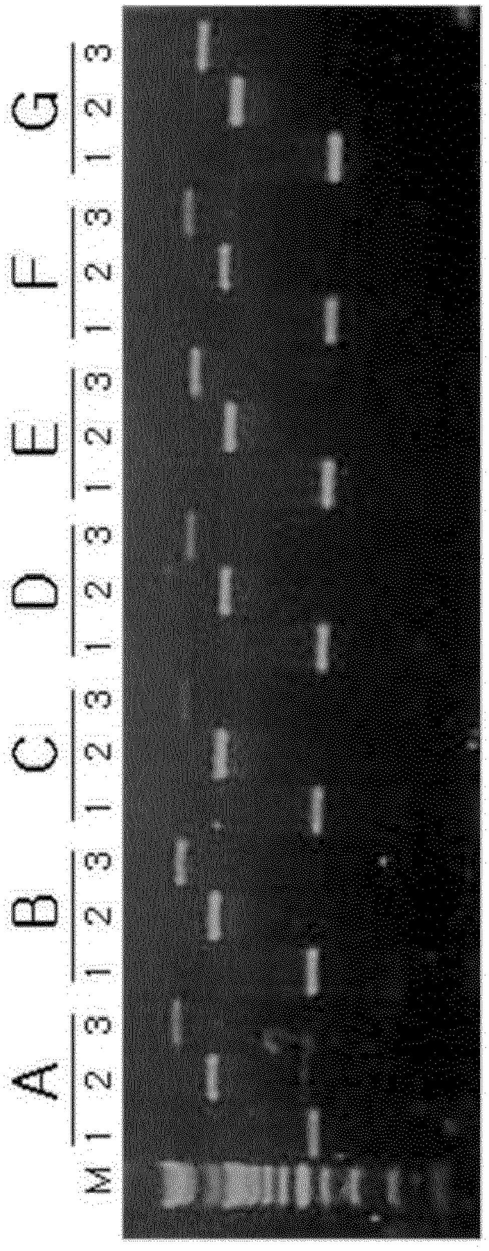

FIG. 13

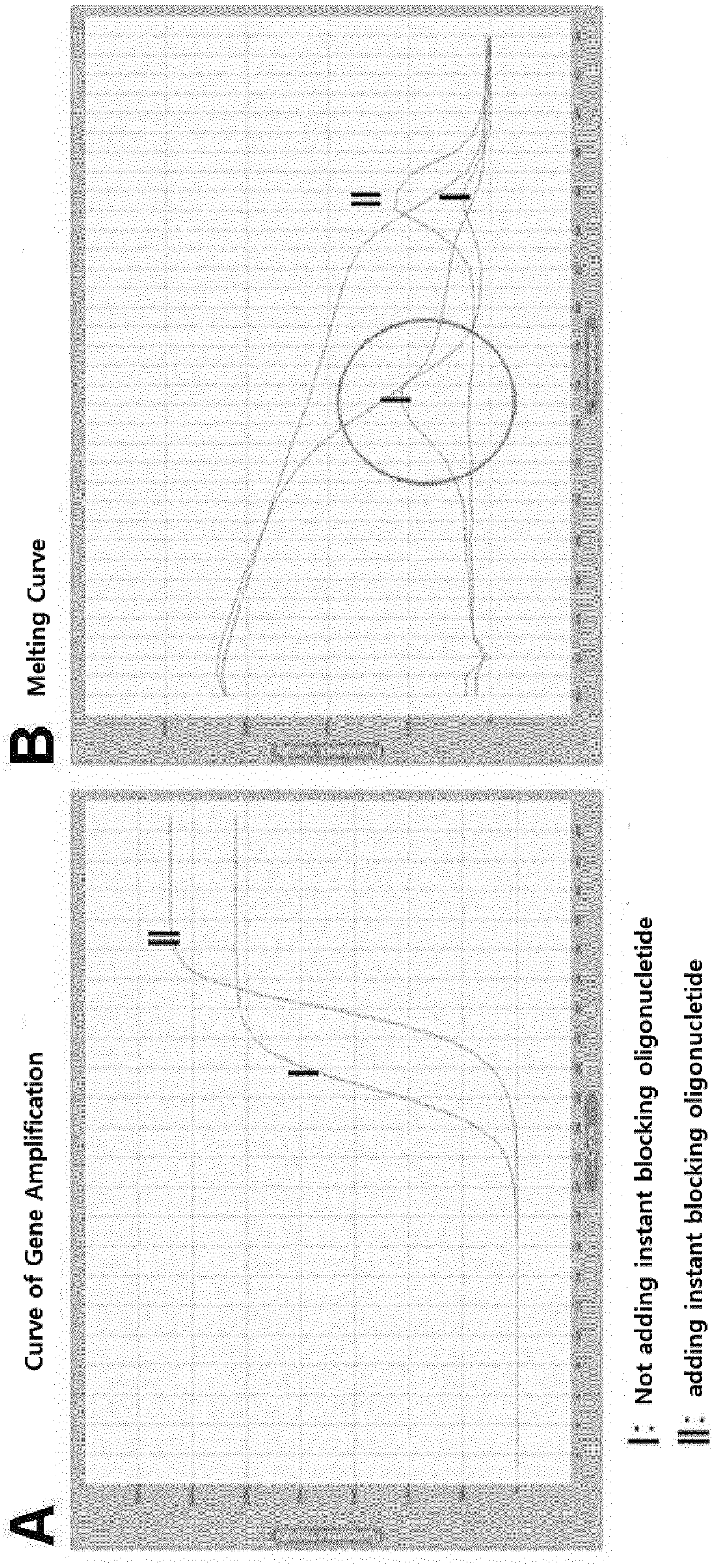
A
Curve of Gene Amplification
B
Melting Curve
I
II
I: Not adding instant blocking oligonucleotide
II: adding instant blocking oligonucleotide

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 101098764
- KR 101098764 B1
- KR 148239
- US 5702590 A
- US 5647994 A
- EP 0691541 A
- US 5336760 A
- US 5897783 A
- US 6187270 B
- KR 102009025743 KR
- WO 9724444 A
- KR 1020100086468 KR
- KR 1020120013757 KR
- KR 10794703
- KR 2008064558 W
- KR 1020120036978
- KR 1020120093222


### Non-patent literature cited in the description


- **MALGOYRE A ; BANZET S ; MOURET C ; BIGARD AX ; PEINNEQUIN A.** *Biochem Biophys Res Commun,* 02 March 2007, vol. 354 (1), 246-52
- **JIYOUNG HONG ; BYUNGHAK KANG ; AHYOUN KIM ; SEOYEON HWANG ; JINHEE AHN ; SUNHWA LEE ; JONGHYEN KIM ; JAE-HAK PARK.** *Doo-Sung Cheon Virol J,* 29 June 2011, vol. 8, 330
- **FUCHS B ; ZHANG K ; ROCK MG ; BOLANDER ME ; SARKAR G.** *Mol Biotechnol,* October 1999, vol. 12 (3), 237-40
- *BMC Mol Biol,* 30 December 2009, vol. 10, 113
- **ESPY M. J. et al.** Realtime PCR in clinical microbiology:application for routine laboratory testing. *Clin. Microbiol. Rev.,* 2006, vol. 19, 165-256
- **BRIAN H. BIRD et al.** *J Clin Microbiol,* November 2007, vol. 45 (11), 35063513
- **MALGOYRE A et al.** *Biochem Biophys Res Commun,* 02 March 2007, vol. 354 (1), 246-52
- **ESPY M. J. et al.** *Clin. Microbiol. Rev.,* 2006, vol. 19, 165-256
- **P A FURMAN ; J A FYFE ; M H ST CLAIR ; K WEINHOLD ; J L RIDEOUT ; G A FREEMAN ; S N LEHRMAN ; D P BOLOGNESI ; S BRODER ; H MITSUYA.** *PNAS,* 01 November 1986, vol. 83 (21), 8333-8337
- **J. J. FURTH ; SEYMOUR S. COHEN.** *Cancer Res,* October 1968, vol. 28, 2061
- **P A FURMAN ; M H ST CLAIR ; J A FYFE ; J L RIDEOUT ; P M KELLER ; G B ELION.** *J. Virol,* October 1979, vol. 32 (1), 72-77
- **CLAIR T ; LEE HY ; LIOTTA LA ; STRACKE ML.** Autotaxin is an exoenzyme possessing 5'-nucleotide phosphodiesterase/ATP pyrophosphatase and ATPase activities. *J. Biol. Chem.,* 1997, vol. 272 (2), 996-1001
- *Biochem. Biophys, Res. Commun,* 1998, vol. 248, 200-203

Sequence Listing

<110> BIONEER CORPORATION

<120> High-sensitivity nucleic acid preparation methods for the detection of nucleic acid by nucleic acid polymerization

<130> PP-B1212

<150> KR10-2012-0036978
<151> 2012-04-09

<150> KR10-2012-0093222
<151> 2012-08-24

<160> 11

<170> KopatentIn 2.0

<210> 1
<211> 23
<212> DNA
<213> Artificial Sequence

<220>
<223> primer

<400> 1
gaaggtgaag gtcggagtca acg 23

<210> 2
<211> 24
<212> DNA
<213> Artificial Sequence

<220>
<223> primer

<400> 2
agtccttcca cgataccaaa gttg 24

<210> 3
<211> 20
<212> DNA
<213> Artificial Sequence

<220>
<223> primer

<400> 3
tagaagcatt tgcggtggac 20

<210> 4
<211> 20
<212> DNA
<213> Artificial Sequence

<220>
<223> primer

<400> 4
aggattccta tgtgggcgac 20

<210> 5
<211> 20
<212> DNA
<213> Artificial Sequence

<220>
<223> primer

<400> 5
tagaagcatt tgcggtggac 20

<210> 6
<211> 22
<212> DNA
<213> Artificial Sequence

<220>
<223> primer

<400> 6
cgtggaagga ctcatgacca ca 22

<210> 7
<211> 20
<212> DNA
<213> Artificial Sequence

<220>
<223> primer

<400> 7
gccttggcag cgccagtaga 20

<210> 8
<211> 23
<212> DNA
<213> Artificial Sequence

<220>
<223> primer

<400> 8
ctgtggatgg cccctccggg aaa 23

<210> 9
<211> 20
<212> DNA
<213> Artificial Sequence

<220>
<223> primer

<400> 9
gacgtccgca tcttgaattg 20

<210> 10
<211> 20
<212> DNA
<213> Artificial Sequence

<220>
<223> primer

<400> 10
ttcccacgat taggagcaca 20

<210> 11
<211> 17
<212> DNA
<213> Artificial Sequence

<220>
<223> primer

<400> 11
caattcaaga tgcggac 17